# EUROPEAN PATENT SPECIFICATION

(11) **EP 1 017 562 B1**
(45) Date of publication and mention of the grant of the patent: **20.06.2007**
(21) Application number: 98950607.6
(22) Date of filing: 03.09.1998
(51) Int. Cl.: B32B 3/06, B32B 3/02, A44B 18/00, D04H 11/08

(54) **LOOP MATERIAL, ITS MANUFACTURE, AND ITS USE IN PRODUCTS**
SCHLINGENARTIGES MATERIAL, HERSTELLUNGSVERFAHREN UND DESSEN VERWENDUNG
MATIERE A BOUCLETTES, SON PROCEDE DE FABRICATION ET SON UTILISATION DANS DES PRODUITS

(30) Priority: 03.09.1997 US 922292
(43) Date of publication of application: 12.07.2000
(73) Proprietor: VELCRO INDUSTRIES B.V., Curacao (AN)
(72) Inventor: SHEPARD, William, H., Amherst, NH 03031 (US); ERICKSON, Paul, R., New Boston, NH 03070 (US)
(74) Representative: Müller-Boré & Partner Patentanwälte
(86) International application number: PCT/US1998/018401
(87) International publication number: WO 1999/011452

(56) References cited:
- EP-A- 0 258 015
- EP-A- 0 765 616
- WO-A-96/14459
- US-A- 3 674 618
- US-A- 3 689 353
- US-A- 3 694 867
- US-A- 4 379 189
- US-A- 4 424 250

## Description

This invention relates to loop material, particularly to material to be engaged with hooking members to form a fastening, to its manufacture and use, and to fasteners comprising such loop material.

In the production of woven and non-woven materials, it is common to form the material as a continuous web that is subsequently spooled. In woven and knit loop materials, loop-forming filaments or yarns are included in the structure of a fabric to form upstanding loops for engaging hooks. As hook-and-loop fasteners find broader ranges of application, especially in inexpensive, disposable products, some forms of non-woven materials have been suggested to serve as a loop material to reduce the cost and weight of the loop product while providing adequate closure performance in terms of peel and shear strength. Nevertheless, cost of the loop component has remained a major factor limiting the extent of use of hook and loop fasteners.

### Summary of the Invention

We have realized that non-woven fabrics constructed with certain structural features are capable of functioning well for their intended purpose as hook-engageable loop fabrics, while providing particular advantage in regard to expense of manufacture and other properties.

According to one aspect of the invention, a loop fastener component of a hook-and-loop fastener, comprising a non-woven web of entangled fibers having a generally planar web body from at least one face of which fibers in the form of hook-engageable loops extend, is characterized in that the web, including the web body and the loops, has weight less than about 4 ounces per square yard (135 grams per square meter), that the web body comprises a non-uniform distribution of fibers in which fibers are in relatively high concentrations in regions of bases of the hook-engageable loops, and in relatively lower concentrations in regions lying between the bases of the loops, and that a substantial number of fibers in regions of lower concentration are taut in the plane of the web body, and extend in different directions radiating from the bases of the loops.

Various embodiments have one or more of the following features:

The bases of the loops contain taut portions of fibers of the web body trained about loop-forming fibers;

Taut fibers of the bases contribute to the definition of free-standing formations that extend from the plane of the web body and contain the hook-engageable loops;

The free-standing formations have elongated trunks from each of which multiple hook-engageable loops extend;

The relatively high concentrations of fibers at the bases of hook-engageable loops define tightened fiber entanglements;

The combined weight of the web body and the loops is less that about 2 ounces per square yard (68 grams per square meter); and

The loops are disposed across the face of the web body in a relatively random pattern.

In some embodiments, a solidified fluid binder is concentrated at the bases of the loops. The binder may be selected from the group consisting of acrylics, urethanes, polyvinyls, formaldehydes, glyoxals and epoxies. Preferably, the solidified fluid binder is configured at the bases of the loops at least partially as a result of capillary flow prior to solidification. In some cases, the solidified fluid binder composes between about 20 and 40 percent (preferably, about one third) of the total weight of the web, including the loops.

In some embodiments, the loop fastener component is combined with a layer of thermoplastic material, such that a second face of the web body, opposite the face from which hook-engageable loops extend, is encapsulated in the layer of thermoplastic material. Preferably, hook elements are integrally molded on a surface of the layer of thermoplastic material.

Various embodiments have one or more of the following features:

Thermoplastic fibers of the web are heat-fused at the bases of hook-engageable loops;

The loop fastener component is formed from a needled non-woven starting batt of given dimension, with the web of the loop fastener component, in its general plane of extent, in a stabilized stretched condition of at least 20 percent (preferably, at least 50 percent and more preferably, at least 100 percent) greater area than the area of the starting batt;

The web is in a stretched condition of at least 20 percent in at least a first direction in the general plane of extent of the web; and

The web is in a stretched condition of at least 20 percent in each of two orthogonal directions in the general plane of extent of the web.

In some embodiments, the starting batt comprises layers of generally parallel fibers with fibers of some layers oriented at acute angles with respect to fibers of other layers, and, as a result of stretching, the acute angles between fibers of the web body corresponding to different layers of the starting batt are generally greater than corresponding angles in the starting batt, while at least many of the fibers corresponding to each layer remain relatively parallel.

In some cases, the layers of a longitudinally extending starting batt extend predominantly transversely across the batt, and, as a result of longitudinal stretching of the starting batt, the acute angles between fibers of the web body corresponding to different layers of the starting batt are generally greater than corresponding angles in the starting batt.

Various embodiments contain one or more of the following features:

The loop fastener component has an overall thickness, including the web body and a majority of the loops, of less than about 0.150 inch (4.0 mm), preferably less than about 0.100 inch (2.5 mm);

The hook-engageable loops extend from associated loop bases within the plane of the web to an average loop height, measured as the perpendicular distance from the web body, of between about 0.020 and 0.060 inch (0.5 and 1.0 mm);

The web has an overall thickness, including the web body and a majority of the loops, the average loop height being between about 0.5 and 0.8 times the overall thickness of the product;

The loop fastener component has between about 50 and 1000 tightened fiber entanglements per square inch (8 and 160 per square cm) of web area, from which hook-engageable loops extend;

The web is generally composed of fibers having a tenacity of at least 2.8 grams per denier (preferably, at least 5.0 grams per denier and more preferably at least 8.0 grams per denier);

The loops extend from the web body to varied heights to form a multi-level arrangement of hook-engageable loops;

The fibers are of a material selected from the group consisting of polyester, polyurethane, polypropylene polyethylene, nylon, homopolymers, mixtures, copolymers, alloys, or co-extrusions thereof and natural fibers;

The loop fastener component has a Gurley stiffness of less than about 300 milligrams; and

The loop fastener component includes separated strengthening strands extending at least in one direction within the plane of the web body.

According to another aspect, the invention provides an enclosure comprising a first closure portion having loops, and a second closure portion having hooks constructed to engage the loops of the first closure portion to hold the enclosure in an open or closed position, the enclosure being characterized in that the first closure portion comprises the above-described loop fastener product.

In some embodiments, the first and second closure portions have a combined overall thickness, engaged and at rest, of less than about 0.075 inch (1.9 mm).

According to another aspect, a disposable article of clothing is provided. The article of clothing has a fabric and a fastener with hooking elements arranged to engage loops of the fabric to form a releasable fastening to retain the article of clothing on a wearer, the article of clothing being characterized in that the fabric comprises the above-described loop fastener component.

According to another aspect, an air filter is provided, characterized in that the filter comprises the above-described loop fastener product arranged to intercept and filter a flow of air.

According to other aspects, geotextile barriers are provided. In one aspect, the barrier comprises the above-described loop fastener product arranged to intercept and filter a flow of groundwater. In another aspect, the barrier comprises the above-described loop fastener product arranged to stabilize a soil layer boundary.

According to another aspect of the invention, a fastener product has a sheet-form polymer base with hooks integrally molded with and extending from one side of the base, and a loop material permanently bonded to and extending over an opposite side of the base. The loop material of the product comprises the above-described loop fastener material, with the loops of the loop fastener material adapted to be engaged by the hooks of the product. The product may be formed, for instance, by use of the techniques described in U.S. Patent 5,518,795.

The invention also provides, in combination, a hook fastener product having hooks extending from a surface thereof, and the above-described loop fastener product, the hooks of the hook fastener product being adapted to engage the loops of the loop fastener product to form a releasable fastening.

In some particularly advantageous embodiments of the combination, the hook fastener product and the loop fastener product have a combined overall thickness, engaged and at rest, of less than about 0.075 inch (1.9 mm), preferably less than about 0.050 inch (1.9 mm).

By "hook-engageable" and similar terms used above and throughout this specification, we mean that the loop material defines openings of size adequate to receive the tip or head portion of a male fastener element (such as a hook-shape or mushroom-shape element, for instance) for forming a fastening.

By the word "entanglements" we mean that the nodes at which a multiplicity of fibers are intertwined in the non-woven web. These entanglements may be relatively loose, as formed directly by a needling process, for instance, or tightened after formation of the entanglements. By the word "knots" we mean entanglements that have been tightened by applying tension to their intertwined fibers in at least one direction in the plane of the web, and remain in an at least partially tightened state.

By "stabilized", we mean that the web is processed to generally maintain its planar dimensions. In other words, a web "stabilized" in a stretched condition will generally maintain its stretched dimensions and not significantly relax or stretch further under conditions of normal use. One way of "stabilizing" the web, for instance, is by solidifying binder at a significant proportion of its entanglements.

We have also realized that such loop fabrics as just described are advantageously produced by employing certain manufacturing techniques and methods.

An important aspect of the invention is a method of forming a loop fastener component for hook-and-loop fastening from a generally planar non-woven batt of entangled fibers. The method is characterized by stretching the batt in at least one direction, thereby producing a stretched web of weight less than about 4 ounces per square yard (135 grams per square meter) and having a generally planar web body with a non-uniform distribution of fibers, with fibers in relatively high concentrations in regions of bases of hook-engageable loops extending from the web body, and in relatively lower concentrations in regions of the web between bases of the loops, with a substantial number of fibers in regions of lower concentration being taut in the plane of the web body, and extending in different directions radiating from the bases of the loops; and thereafter stabilizing the web in its stretched condition.

Various embodiments of the method of the invention have one or more of the following characterizations:

The stretching causes taut fibers of the web body to be trained about loop-forming fibers in the bases of the loops;

The batt is stretched in a manner that the fibers of the bases contribute to the definition of free-standing formations that extend from the plane of the web body and contain the hook-engageable loops;

The batt is stretched in a manner that the formations comprise elongated trunks from each of which multiple hook-engageable loops extend;

The batt is stretched in a manner that the relatively high concentrations of fibers at the bases of hook-engageable loops define tightened fiber entanglements;

The batt is stretched so as to produce a web having a weight, including the loops, of less than about 2 ounces per square yard (68 grams per square meter); and

The loops become disposed across the face of the web body in a relatively random pattern as the batt is stretched.

In some presently preferred embodiments of the method, the web is stabilized in its stretched condition by solidifying a fluid binder, applied to the web body under conditions that concentrate the binder at the bases of the loops.

The fluid binder may be selected, for instance, from the group consisting of acrylics, urethanes, polyvinyls, formaldehydes, glyoxals and epoxies.

Preferably, fluid binder is applied under conditions enabling the fluid binder to be configured at the bases of the loops, at least partially as a result of capillary flow prior to solidification. In many cases, the fluid binder is applied prior to at least some of the stretching.

Various embodiments of the method of the invention have one or more of the following characterizations:

A selected amount of binder is applied, such that the binder composes between about 20 and 40 percent (preferably, about one third) of the total weight of the web, including the loops;

The batt is provided with fusible thermoplastic fibers embedded therein, the stretched web being stabilized under conditions which cause the fusible thermoplastic fibers to become heat-fused at the bases of hook-engageable loops;

The web is stabilized in a stretched condition in which the web has, in its general plane of extent, an area at least 20 percent (preferably, at least 50 percent and more preferably, at least 100 percent) greater than the area of the batt prior to stretching;

The batt is stretched at least 20 percent in a first direction in the general plane of extent of the web body;

The batt is stretched at least 20 percent in each of two generally orthogonal directions in the general plane of extent of the web body;

The starting batt comprises layers of generally parallel fibers with fibers of some layers oriented at acute angles with respect to fibers of other layers, the product characterized in that, as a result of stretching, the acute angles between fibers of the web body corresponding to different layers of the starting batt are generally greater than corresponding angles in the starting batt, while at least many of the fibers corresponding to each layer remain relatively parallel.

The layers of a longitudinally extending starting batt extend predominantly transversely across the batt, and the batt is stretched longitudinally under conditions which increase the acute angles between fibers of the web body corresponding to different layers of the starting batt.

In some embodiments, the stretched web is combined with a layer of thermoplastic material, such that a second face of the web body opposite from the face from which hook-engageable loops extend, becomes encapsulated in the layer of thermoplastic material. Preferably hooks are molded integrally with an exposed surface of the layer of thermoplastic material.

According to another aspect of the invention, a method of forming a loop fastener component for hook-and-loop fastening from a generally planar non-woven batt of entangled fibers is characterized by first stretching the batt in at least one direction, thereby producing a stretched web having a generally planar web body with a non-uniform distribution of fibers, with fibers in relatively high concentrations in regions of bases of hook-engageable loops extending from the web body, and in relatively lower concentrations in regions of the web between bases of the loops, with a substantial number of fibers in regions of lower concentration being taut in the plane of the web body, and extending in different directions radiating from the bases of the loops; and then stabilizing the web in its stretched condition.

In some cases, the web is stabilized in its stretched condition by solidifying a fluid binder, applied to the web body under conditions that concentrate the binder at the bases of the loops.

According to another aspect of the invention, a method is provided for filtering air, characterized by arranging the web of the above-described loop fastener product to intercept a flow of air to be filtered.

According to another aspect, a method of filtering groundwater is provided, characterized by arranging the web of the above-described loop fastener product to intercept a flow of groundwater to be filtered, and by securing the web in position by engaging the loops of the loop fastener product with a length of hook fastening tape.

According to another aspect of the invention, a method of stabilizing soil is provided, characterized by arranging the web of the above-described loop fastener product between adjacent layers of soil to be stabilized, and by securing the web in position by engaging the loops of the loop fastener product with a length of hook fastening tape.

The invention can provide a very inexpensive loop product which can very effectively engage and retain hooks, such as in hook-and-loop fasteners. The loop product can be particularly useful in combination with extremely small, inexpensive molded hooks as fasteners for disposable products, such as diapers, medical devices or packaging. In use as a geotextile barrier, the loop product of the invention is readily secured in place by lengths of hook fastener tape.

### Brief Description of the Drawings

Fig. 1 is an enlarged side view of a hook-and-loop fastener.
Figs. 2 and 3 are enlarged plan and side views, respectively, of a loop fastener product.
Fig. 2A is a plan view of a loop fastener product, enlarged 50X and showing the structure of the web; and Fig. 2B is a schematic view of the structure shown in Fig. 2A.
Fig. 3A is a further enlarged portion of the photograph of Fig. 3; Fig. 3B is a sketch of the structure in the foreground of Fig. 3A; and Fig. 3C is a highly enlarged plan view of a portion of the loop fastener product of Fig. 3.
Fig. 4A is an enlarged side view of a hook-and-loop product made by ultrasonically welding a loop product to a hook product. Fig. 4B illustrates a two-sided fastener product formed with loops on one side and hooks on the other.
Fig. 5 illustrates a machine and process for forming the product of Fig. 4B.
Fig. 6 illustrates a loop material containing longitudinal fibers.
Figs. 7A-7F depict illustrative products incorporating the loop material of Fig. 2; in order, the illustrated products are; the outer fabric of a disposable diaper (Fig. 7A); a disposable surgical gown (Fig. 7B); a box closure (Fig. 7C); a bag closure (Fig. 7D); an air filter (Fig. 7E); and a geotextile barrier (Fig. 7F).
Fig. 8 is a top view of an apparatus for making a nonwoven fabric.
Fig. 9 is a side elevational view of the apparatus of Fig. 8.
Fig. 10 shows an alternative arrangement of the second needling stage of the apparatus of Fig. 9.
Figs. 11 and 11A are plan and side views, respectively, of a batt of needled material after the second needling stage.
Fig. 12 is a schematic view of an apparatus for stretching and stabilizing a nonwoven material.
Figs. 12A and 12B are enlarged views of area 12A in Fig. 12 under two different conditions of operation, and Fig. 12C illustrates another embodiment in which the product is stabilized by fusing fibers.

### Description of Embodiments

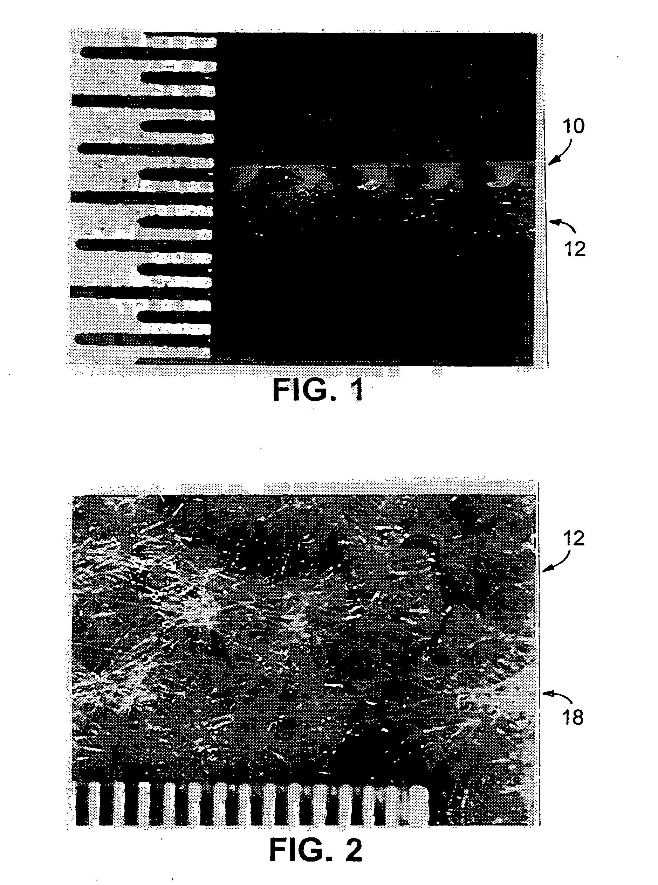

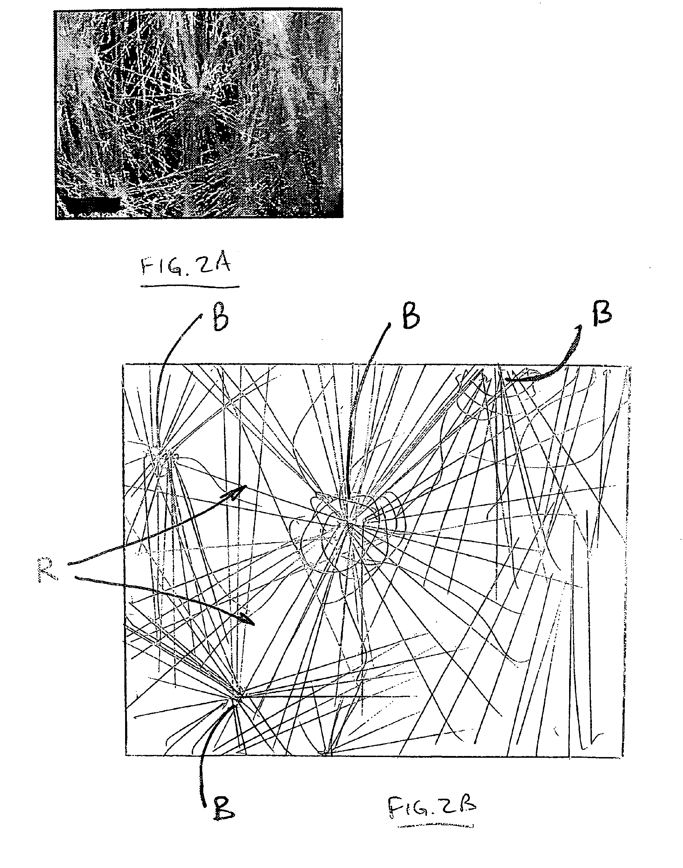

Referring first to Fig. 1, a molded hook fastener product 10 is shown engaging the loops of a very thin loop product 12. The photograph is quite enlarged, as shown by the scale on the left side of the photograph. The minor divisions of the scale each represent a length of 1/64th (0.0156) inch (0.40 mm). Hook product 10 is of the CFM-29 designation, available from Velcro U.S.A Inc. of Manchester, New Hampshire, U.S.A., and has hooks of only 0.015 inch (0.38 mm) height. Referring also to Figs. 2 and 2A, loop product 12, a feature of the present invention, is very thin (as evidenced by the scale of the photographs and its lack of opacity) and has relatively free fibers forming loops extending from one side of a continuous, tangled mat of fibers. In this and the following photographs all scale graduations, unless otherwise marked, are in 0.0156 (1/64) inch (0.40 mm) increments.

As shown in Fig. 2, and especially in Fig. 2A, a substantial number of the fibers of the mat of loop product 12 are taut (i.e., not slack, regionally straight), extending between knots 18 of the loop product fabric. The taut fibers have been straightened by tension applied in at least one direction in the plane of the fabric mat. The individual fibers of the mat follow no definite pattern as in a woven product, but extend in various directions within the plane of the fabric mat. The loops that extend from the loop product are of the same fibers that comprise the mat but extend beyond the general mass of the mat, out of the plane of the mat, generally from associated knots 18. The knot density of the sample shown in the photograph was determined to be approximately 180 knots per square inch by counting the number of visible knots within a given square area. The knots themselves are fairly tight, made up of several monofilament fibers, and are interconnected by the taut fibers seen running between them. In between knots, the thin fiber mat is not very dense and is sheer enough to permit images to be readily seen through it. For low cost application, the fabric preferably weighs less than about 2 ounces per square yard (68 grams per square meter).

In this particular embodiment, the fibers of the mat are held in their taut, straightened condition by a water-based, acrylic binder (not visible in the photograph) applied to the side of the mat opposite the loops to bind the mat fibers in their taut condition to stabilize the areal dimensions of the fabric, and to secure the loops at their associated knots. The binder generally ranges between 20 and 40% of the total weight of the fabric and in the presently preferred embodiments accounts for about one third of the total product weight. The resulting fabric is dimensionally stable and strong enough to be suitable for further processing by standard fabric-handling techniques. The fabric also has a slight stiffness, like a starched felt, which can be mitigated by softeners or mechanical working if desired.

The schematic view of Fig. 2B illustrates the structure of planar web 12, as viewed from one face of the web. In this view, the loop-engageable loops extend out of the plane of the web, from one side. Web 12 is composed of a non-uniform distribution of entangled fibers, with relatively high concentrations of the fibers at the bases, B, of corresponding loop structures, and relatively lower concentrations of the fibers in regions, R, lying between loop bases, B. The relatively high concentrations of fibers at bases B correspond to tightened fiber entanglements. As illustrated in this sketch, and visible in Fig. 2A, a substantial number of the fibers in the regions, R, between loop bases are taut in the plane of the web, extending in different directions radiating from loop bases, B. By "taut", we mean that a large percentage of these inter-base fibers have no give or slack, such that they may transmit an applied tensile force with little or no displacement. We believe that the taut fiber portions extending across the sparse regions between loop bases account for some of the beneficial properties of the loop product, giving it a perceptibly high strength-to-weight ratio as a fastener component.

Near the center of both Fig. 2A and Fig. 2B is a particularly visible loop base B, from which taut fibers can be seen emanating in a radial pattern. Also note that there are some fibers which are wrapped at least partially about other fibers of the loop base. These wrapping fibers are the result of stretching, during which straightening fibers encounter loop fibers extending through the planar web. As the web is further stretched, the loop fibers provide obstructions about which the straightening web base fibers are trained as they are displaced within the web plane. Thus the bases, B, of the loop structures contain both portions of the loop-forming fibers extending out of the plane of the web and trained portions of taut fibers lying generally in the plane of the web. The trained portions of the taut fibers within the loop bases therefore contribute, as the web is stretched, to the definition of the free-standing loop formations. When the web is stabilized by binder, for instance, these bases B become relatively rigid nodes and, importantly, provide anchoring for their associated loops. Thus the stretched and stabilized web, in some respects, resembles a planar truss, with its taut radiating fibers forming tensile members between base nodes. As the taut fibers may be readily "bent" out of their plane as the web flexes, the structure retains an advantageously high flexibility while resisting elongation and shrinkage within its original plane.

The individual fibers of loop fabric 12 shown in Fig. 2 have low denier and substantial tenacity (i.e., tensile strength per unit diameter) to work with very small hooks such as those illustrated in Fig. 1. Fibers with tenacity values of at least 2.8 grams per denier have been found to provide good closure performance, and fibers with a tenacity of at least 5 or more grams per denier (preferably even 8 or more grams per denier) are even more preferred in many instances. In general terms for a loop-limited closure, the higher the loop tenacity, the stronger the closure. The fibers of fabric 12 of Figs. 1 and 2 are 6 denier staple polyester fibers (cut to four inch lengths) and as a result of the method of the manufacture, are in a drawn, molecular oriented state, having been drawn with a draw ratio of at least 2:1 (i.e., to at least twice their original length) under cooling conditions that enable molecular orientation to occur, to provide a fiber tenacity of about 3.6 grams per denier. The fibers in this example are of round cross-section and are crimped at about 7.5 crimps per inch (3 crimps per cm). Such fibers are available from E.I. Du Pont de Nemours & Co., Inc., in Wilmington, Delaware under the designation T-3367 PE T-794W 6x4. The loop fiber denier should be chosen with the hook size in mind, with lower denier fibers typically selected for use with smaller hooks. For low-cycle applications for use with larger hooks (and therefore preferably larger diameter loop fibers), fibers of lower tenacity may be employed.

As an alternative to round cross-section fibers, fibers of other cross-sections having angular surface aspects, e.g. fibers of pentagon or pentalobal cross-section, can enhance knot tightening for certain applications. Regardless of the particular construction of the individual fibers, they, are selected to have a surface character that permits slippage within the knot-forming entanglements during tightening so as to enable stretching the batt without undue fiber breakage.

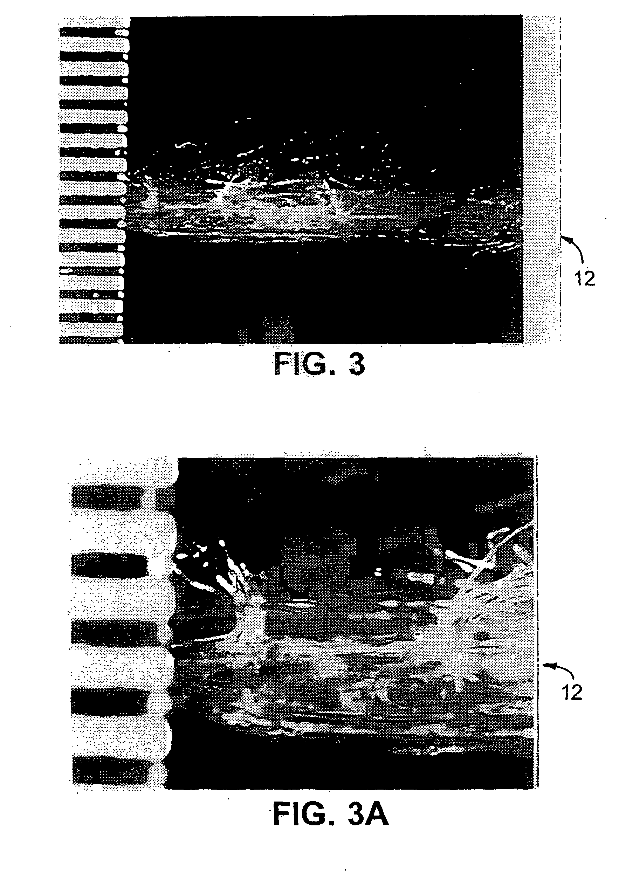

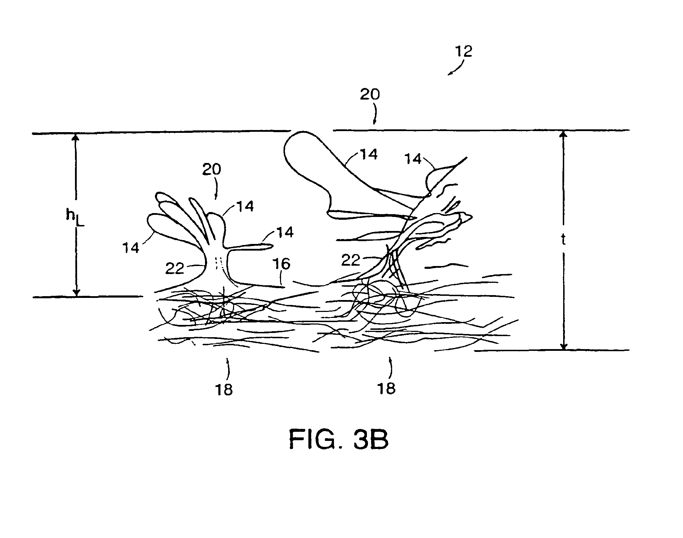

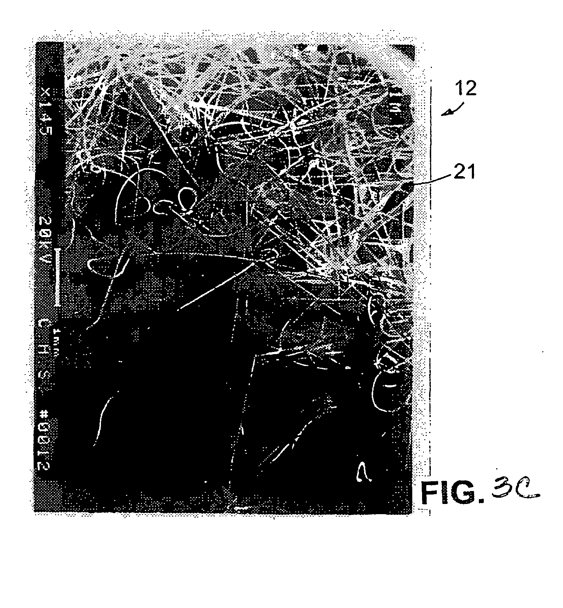

Referring to Figs. 3, 3A and 3B, the loops 14 of loop fabric 12 of this embodiment project primarily from one side of the fabric. The stabilizing binder, in this case, is applied to the other side. The loop product is extremely thin for use with very small hooks. The product shown, for instance, works well with hooks of about 0.015 inch (0.4 mm) height and has a loop height h_{L} (i.e., the height of loops 14 from the near general surface of fiber mat 16) of about 0.055 inch (1.40 mm). The loop product has an overall thickness, t, including a majority of the loops, of only about 0.090 inch (2.3 mm). When measuring loop height in products without a visibly distinguishable upper mat surface, we define the near surface of the mat to be the lowest planar surface above about 80 percent of the total mass of fibers. The loops preferably vary in height for good engagement, and the average loop height should generally be greater than the height of the hooks with which the loop product is to be used, and preferably between 2 and 10 times the head height of the hooks used for applications requiring good shear strength. For fasteners which are primarily loaded in peel, or by loads perpendicular to the plane of the base, the loops may be up to 15 times the head height of the hooks. For example, for use with 0.015 inch (0.4 mm) CFM-29 hooks (which have a head height of 0.006 inch or 0.15 mm), the average height h_{L} of the loops should be between about 0.012 and 0.060 inch (0.3 and 1.5 mm) for good shear performance. For use with 0.097 inch (2.5 mm) CFM-24 hooks (which have a head height of 0.017 inch or 0.43 mm and are also available from Velcro U.S.A. Inc.), the average height of the loops should be at least 0.035 inch (0.89 mm) and may be as high as 0.250 inch (6.4 mm) for applications focusing on peel loading. For low cost, flexible loop fabrics, the average loop height should generally be between about 0.020 and 0.060 inch (0.5 and 1.5 mm), and should be between about 0.5 and 0.8 times the overall thickness, t, of the loop product.

As seen in Figs. 3A and 3B, loops 14 extend from free-standing clusters of loop fibers extending from the fibrous mat 16. The clusters 20 which have several monofilament loops 14 extending from a common elongated, substantially vertical trunk 22 we call "loop trees". Another example of a "loop tree" is seen in Fig. 3C. Each loop tree 20 extends from a corresponding knot 18 in which the loops of the cluster are anchored. Interstices between individual filaments in the trunk portion 22 of each tree or at the base of each bush, and in each knot 18 provide paths for the wicking of liquid binder, under the influence of surface tension of the liquid binder, to provide additional localized stiffness and strength. Importantly, the density of clusters in the plan view is very low (Figs. 2 and 2A), leaving sufficient room between the "branches" of neighboring trees to accommodate hooks and deflected loop material during engagement.

Accumulations of solidified binder 21 can be seen in the knots in the highly magnified plan view of Fig. 3C. Applied in liquid form in this example, preferably before the knots are tightened, the binder contributes to securing the loops against being pulled out of the web.

Referring back to Fig. 1, with proper clearance between loops for the accommodation of hooks, the fully engaged fastener (i.e., the loop product and mating hook product together) has an overall thickness of only the sum of the thickness of the hook product (including hooks) and the "ground" portion of the loop product (i.e., the thickness of the mat 16 between loop clusters, Fig. 3B). In other words, the free standing loops of the loop product do not add to the thickness of the completed fastener. Because of the ultra-thin ground portion 16 of the loop product disclosed herein (see Fig. 3B), the combination of loop product 12 with mating hook product 10 provides a fastening of very small thickness. For example, the engaged fastener of Fig. 1 has an overall thickness of only about 0.050 inch (1.3 mm; thinner, in this case, than the overall thickness of the unengaged loop product, as the taller loop clusters are somewhat compressed by the hook product engaged with shorter loop clusters).

In addition to being advantageously thin, loop fabric formed according to the new principles is particularly flexible. Flexibility can be very important in some fastener applications, especially when the fastener must flex during use, as when used on an article of apparel. In such instances, the loop product of the invention should have a bending stiffness of less than about 300 milligrams, preferably less than about 100 milligrams, as measured with a Gurley type tester. More details on the use of Gurley type testers can be found in Method T 543 OM-94, published in 1984 by the Technical Association of Pulp and Paper (TAPPI).

Various synthetic or natural fibers may be employed in the invention. In some applications, wool and cotton may provide sufficient fiber strength. Presently, thermoplastic staple fibers which have substantial tenacity are preferred for making thin, low-cost loop product that has good closure performance when paired with very small molded hooks. For example, polyolefins (e.g., polypropylene or polyethylene), polyesters (e.g., polyethylene terephthalate), polyamides (e.g., nylon), acrylics and mixtures, alloys, copolymers and coextrusions thereof are suitable. Polyester is presently preferred.

For a product having some electrical conductivity, a small percentage of metal fibers may be added. For instance, loop products of up to about 5 to 10 percent fine metal fiber, for example, may be advantageously employed for grounding or other electrical applications.

Various binders may be employed to stabilize the fabric. By "binder" we mean a material within the mat (other than the fibers forming the main fastener loops) that secures the loop fibers at associated knots. In some applications, the binder is an adhesive. In other applications, the binder is in the form of fibers of low-melt polymer dispersed throughout and entangled within the fabric. These low-melt fibers are melted to wet the knot-forming entanglements and then cooled and solidified to secure the loops and stabilize the fabric. The binder preferably fully penetrates and permeates the interstices between individual fibers in the entanglements of the mat. When employing a liquid binder, the binder is preferably selected to have a sufficiently low viscosity and surface tension to enable it to flow into the untightened (or tightening) entanglements. In the embodiments in which the entanglements are subsequently tightened (such as the loop product shown in Fig. 2), this selected distribution of the fluid binder helps to secure the knots with minimal stiffening of the overall product and without requiring substantial amounts of binder.

In any event, the amount and penetration of the binder should be selected to avoid substantial interference with the desired hook-engaging function of the loops while adequately stabilizing the mat and securing the loops against being pulled from their associated entanglements. For use in applications in which the loop product may come in direct contact with sensitive skin, such as in diapers, the amount and type of binder should also be selected to be biocompatible to avoid skin irritation. As irritation can be aggravated by stiffness, preferably only enough binder to perform the above functions is applied. In some applications, for instance those in which the loop product is directly adhered to a supporting fabric and which does not require substantial fastener strength, the loop product may be provided without a binder.

In important instances, the binder also includes an organic or inorganic fire-retardant, such as antimony oxide, zinc borate, aluminum trihydrate or decabromobiphenyl oxide.

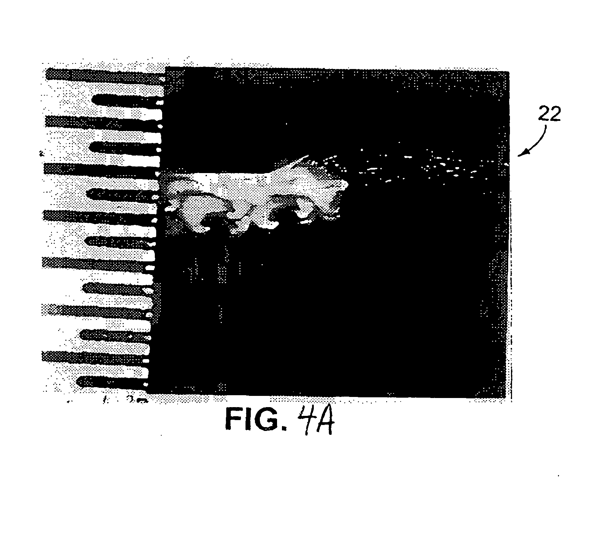

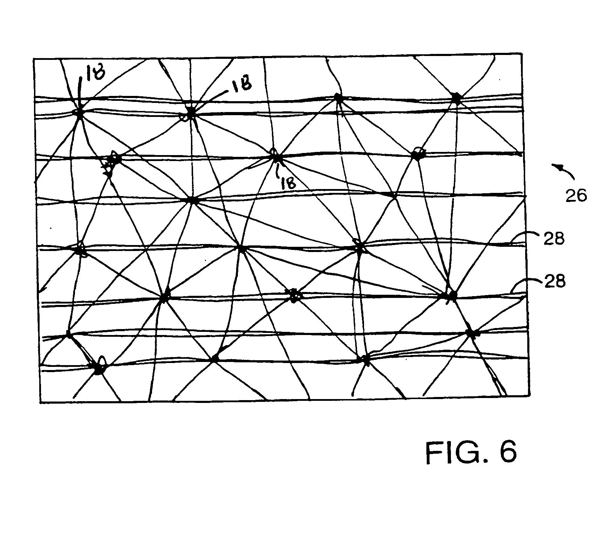

The specific loop product 12 of Figs. 1 and 2 includes about one third by weight water-based acrylic binder produced by mixing 80 parts "NACRYLIC" X-4280, a self-reactive acrylic emulsion, with 20 parts "X-LINK" 2804, a self-crosslinking, polyvinyl acetate/acrylate emulsion, both available from National Starch and Resin Company in Bridgewater, New Jersey. As produced, loop product 12 substantially consists only of the drawn fibers of the thin mat, some of which extend out of the mat to form loops, and the binder. Without any additional backing or laminate, it is strong enough to be handled as a fabric material, and may be applied to surfaces as a closure member by sewing, ultrasonic welding, adhesive, radio frequency welding, or other known attachment means. Fig. 4A, for instance, shows a hook-and-loop product 22 formed by ultrasonically welding a piece of the loop material 12 of Fig. 2 to a piece of CFM-29 hook product. The resulting product 22 can be formed into a closed band by engaging its loops with its hooks. Fig. 5B is an enlarged view of stitching 24 securing the loop product to a substrate.

Referring to Fig. 6, another loop fabric material 26 includes, in addition to the drawn, molecularly oriented, randomly laid fibers previously described, continuous robust longitudinal monofilament fibers 28 extending substantially in one direction to augment the tensile strength of the finished fabric in the direction of the strands. For this purpose the diameter of the longitudinal monofilaments is selected to be larger than the barbs of the needles to reduce engagement of the monofilaments by the needles during the needling process. Monofilaments 28 are preferably crimped to enable them to be stretched a limited amount in the machine direction as the fabric is stretched before being stabilized. Alternatively, a stretchable scrim of similarly large fibers or film may be incorporated into the web of fibers to increase tensile strength in both longitudinal and lateral directions.

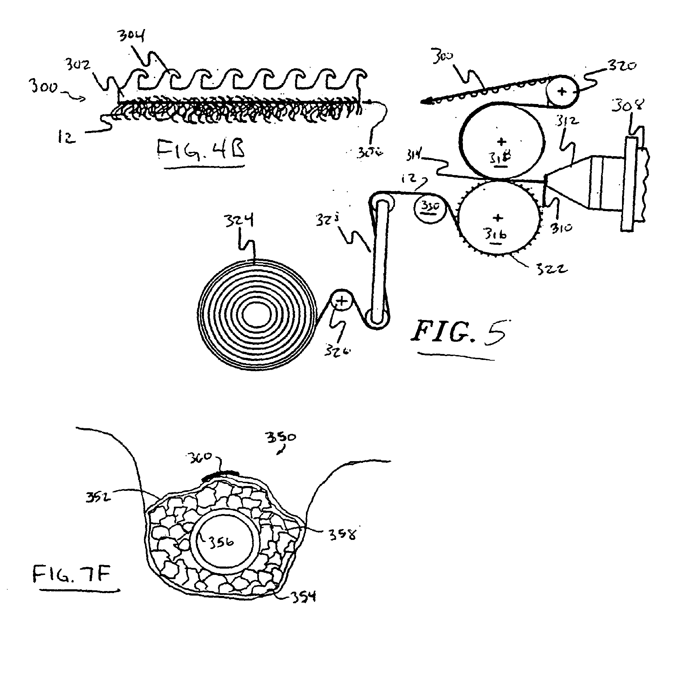

Fig. 4B illustrates a one-piece fastener product consisting of a lamination of the above-described loop material and molded hook tape. Product 300 has a base 302 with integrally molded hooks 304 projecting from one side and the above-described non-woven loop material 12 secured to the other side. At the interface 306 between the two layers the plastic from base 302 flows around and entraps some of the fibers of the base web of loop material 12, encapsulating one face of the web in thermoplastic material to form a permanent laminate of the two layers. Because of the extremely light nature of the non-woven material of the invention, care must be taken to only encapsulate the web and to leave the functional loops exposed for engagement with hooks 304. The properties of the non-woven material, the viscosity of the plastic and the pressure in the nip (see Fig. 5) will determine the degree to which the plastic flows into the fibrous network, or put alternately, the degree to which the non-woven will imbed into the plastic. The resulting laminated product is particularly thin and flexible, due in part to the thinness of the loop material.

The product of Fig. 4B may be economically formed by the process and apparatus illustrated in Fig. 5. Extruder barrel 308 melts and forces the molten plastic 310 through die 312 into the nip 314 between base roller 316 and cavity roller 318 containing cavities to form the hooks of a strip fastener of the well known hook and loop type. The strip fastener material formed in nip 314 travels around the periphery of cavity roller 318 and around stripping roller 320, which assists in pulling the finished product 300 from the cavity roll, and from there to a windup device, not shown.

While many methods of feeding sheet material to the forming section of the hook forming device are possible, Fig. 5 illustrates a device particularly well adapted to that purpose. By introducing loop material 12 into nip 314 at the same time molten plastic 310 is forced into the nip, the loop material will bond intimately with the fastener to become an integral part of the structure of the strip fastener. Optionally, a set of pins 322 at the edges and around the periphery of backing roller 316 carry the loop material 12 into nip 6 in a flat, unwrinkled state. To assure proper tensioning and alignment of the secondary sheet material, a roll 324 of loop material 12 is mounted on a let off device and threaded around diversion roller 326 into a web straightening device 328, well known in the art as typically sold by the Fife Manufacturing Company which assures that the web of loop material is centered as it is fed onto backing roller 316 around scroll roll 330, which has ribs of elastomeric material to firmly grip the sheet and impinge it against backing roller 316 and onto pins 322. Pins 322 and roller 316 deliver the web into nip 314 along with molten plastic 310. As molten plastic 310 is forced by the pressure imposed upon it by the narrow space of nip 314, it flows into cavities in cavity roller 318 and also into pores in the adjacent face of the loop material being carried by backing roller 316. In this way the loop material is intimately joined to the base of the forming hook fastener tape to form laminated product 300.

For more detail about proper operation of the apparatus of Fig. 5, the reader is referred to U.S. Patent 5,260,015 to Kennedy, et al., which discloses laminates made with heavier loop materials.

The very low thickness and stiffness of the above-described loop material, along with its low cost and good closure performance, make it a particularly useful component of many other products, as well.

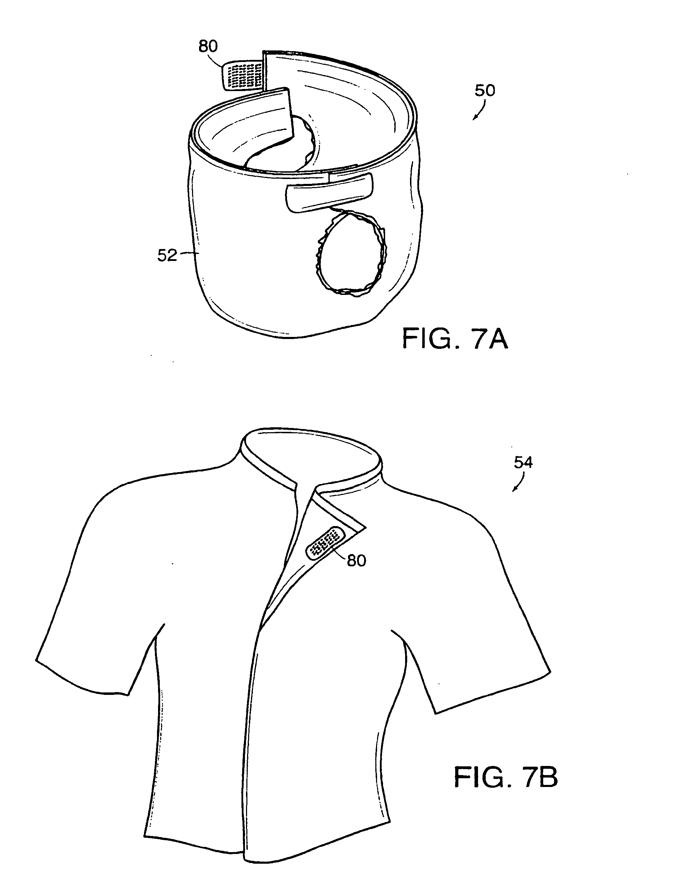

Referring to Fig. 7A, a diaper 50 has an outer shell 52 fabricated from the above-described loop product, such that it can be engaged by hooks 80 anywhere over a significant proportion of its surface, while providing the outer "wearing" surface of the article.

In another preferred embodiments, not shown, a panel or patch of the loop product is laminated (directly, or via a carrier sheet,) to the outer liner of a diaper, to provide a suitably large "landing" area that hooks can engage. In one advantageous instance, a panel of the loop product is laminated to a carrier film, which, on its back, has a layer of pressure-sensitive adhesive for automated application to diaper liners or the like during manufacture of the diaper.

Fig. 7B shows a surgical gown 54 fabricated from the loop material of the invention and capable of being "hooked" by hooks 80 at any point over a large portion of its surface. As with the above-described diaper constructions, as an alternative a panel or patch of the loop product may be attached to the outer surface of a surgical gown.

In such applications in which the products are considered disposable after single use, the loop material only need withstand a relatively small number of hooking cycles (e.g., 3 to 5) over the product's useful life. We refer to these as "low cycle" applications. Loop products in this category may be fabricated by stretching needled fabric in excess of 100%, as much as 150% or more.

Other applications, such as strapping, can require the loop to withstand a higher number of cycles and higher stress. These relatively "high cycle", high strength applications generally are achieved by forming loops with higher denier (or higher tenacity) fibers than those suitable for lower performance conditions. For instance, polyester fibers of 15 denier are advantageously employed for high strength high cycle applications employing large hooks such as CFM 15 (0.035 inch or 0.89 mm high) or MV 8 (0.100 inch or 2.5 mm high) or mushroom fasteners, all available from Velcro USA Inc. Loop products in this category may be prepared by stretching in the range of 50 percent to 100 percent stretch, for example.

Fig. 7C shows a box 56 with hook closure strips 58 that engage loop closure strips 60 of our loop product to hold the flaps 62 of the box in a closed position. Loop closure strips 60 have pressure sensitive adhesive backing, by which they are permanently applied to box flaps 62. Supplied as die cut sections on a release liner, loop strips 60 can be applied to the box flaps with a common, automatic label head, for instance. The box is useful for applications where it must be repeatedly opened while its contents are progressively consumed, for example, a pet food box.

There are many different adhesives and backing materials that can be included on the back (i.e., "non-loop") side of the loop product, either in place of or in addition to the binder described above. For instance, a binder adhesive may be formulated to be pressure sensitive, with or without a release liner, such that the final product may be applied to a substrate by the application of pressure. Without a release liner, as the product is rolled or spooled the pressure sensitive backing lies against and adheres to the hook-engageable loops to keep the product in rolled form until unrolled by peeling, leaving a sufficient amount of undisturbed adhesive on the back of the fabric to secure the product to a substrate. Other coatings, such as heat sensitive or "hot-melt" coatings, or water-activated or solvent-activated coatings, may also be used with suitable heating or application of activating fluid at the time of activation to secure the loop material to the item on which a closure is desired.

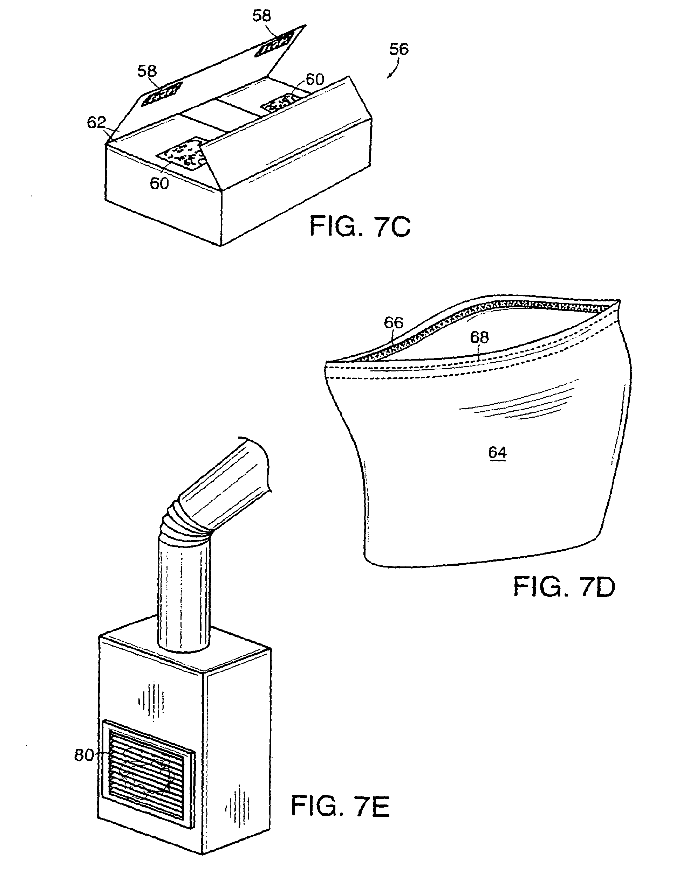

In cases where the fiber material is chosen to be of the same type as, or compatible with, a substrate material upon which it is desired to secure the loop fabric, the loop product may be directly thermally fused to the substrate. For instance, Fig. 7D shows a polyethylene bag 64 to which a polyethylene hook strip 66 and a polyethylene loop strip 68 have been thermally bonded or welded. It can be closed in rapid fashion by simply pressing one end of the closure together by opposed fingers and sliding the fingers from one end to the other of the closure. Such a closure does not require accurate alignment of the mating features to be secured, and can enable some desirable venting and filtration through the secured closure. Such a venting, filtering closure, which is practically enabled by the low cost of the loop product of the invention, is useful for vegetable containers and charcoal briquette bags, for instance. The closure also allows equilibration of the pressure inside and outside of bags or packages constructed to travel in airplane cargo holds.

The loop product may be flame-laminated to other materials, such as to open-cell foam. The flame lamination process not only adheres the loop product to the foam, but stabilizes the foam. Products economically produced in this manner include disposable medical devices, such as limb or joint braces, straps for colostomy bags, and blood pressure cuffs. Other products include merchandise and trade show displays with large surfaces of loop product upon which hooks can be used for mounting various displays.

The low density of the loop material makes it useful as a filter media for filtering relatively high volume flows of air, such as in HVAC systems and the like. Fig. 7E shows the loop material described above, associated with a suitable frame as an inexpensive, replaceable filter 80. Stiffened by suitable proportions of binder and/or a stiffening agent applied to the web, the loop material is preferably permanently corrugated to increase the surface area of the filter intercepting a flow of air and to enhance the rigidity of the filter.

Fig. 7F illustrates an example of the use of the non-woven material of the invention as a geotextile, as a silt screen for a soil drainage system. A trench 350 is formed in the soil at or below the footing level of a foundation, for instance, and a wide, continuous length 352 of non-woven material, formed according to the process described above, is laid along the trench. A layer 354 of stone is placed on the non-woven material 352 in the bottom of the trench, and a perforated or slit drain pipe 356 is laid on top of the layer 354 of stone. The pipe is then covered with another layer 358 of stone to form a highly permeable layer about the exterior of the pipe, the interior of which defines the drain. The layers of stone form open interstices for the free flow of groundwater into the pipe. The edges of the length 352 of non-woven material are then pulled up over the stone and overlapped to form a continuous tube about the stone and pipe. The edges of the non-woven material are held in this overlapped condition by a continuous length of hook fastener product 360 having hooking elements extending from one side for engaging and retaining the fibrous loops of the non-woven material. The trench is subsequently back-filled. The porous structure of the non-woven material 352 enables groundwater to drain from the surrounding soil into the stone and pipe while providing an adequate barrier to sand and silt that would otherwise clog the interstices of the stone layer and the slits in the pipe.

The non-woven material described above may be employed in other geotextile applications, as well. For instance, large sheets of the material may be placed edge to edge over large areas to stabilize the boundary between different soil types. Advantageously, the fastening properties of the above-described material enable adjacent sheets to be easily joined with lengths of hook fastener product. Joining adjacent sheets of ground stabilization material with continuous fastener tape helps to keep the sheets in place and to maintain the continuity of the barrier formed by the non-woven material.

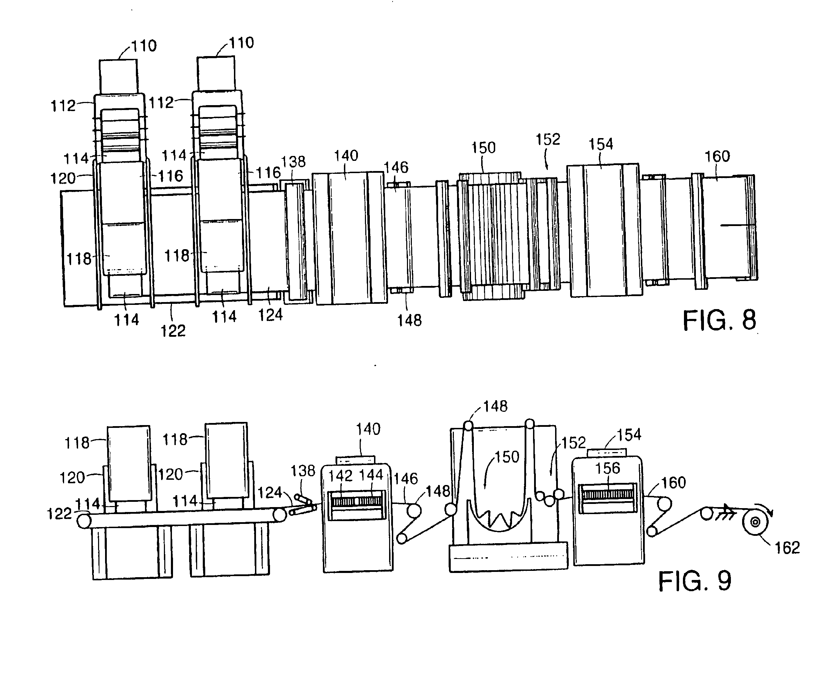

Referring to Figs. 8 and 9, an apparatus for producing the above-described loop material includes a feeder 110 (with, e.g., bale breakers, blender boxes or feed boxes), which feeds staple fibers of a desired length of drawn fibers to carding machines 112. The carding machines 112 card the staple fibers to produce carded webs of fibers 114 which are picked up by the take-off aprons 116 of crosslappers 120. The crosslappers 120 also have lapper aprons 118 which traverse a floor apron 122 in a reciprocating motion. The crosslappers lay carded webs 114 of, for example, about 12 to 18 inches (30 to 45 cm) width and about one inch (2.5 cm) thickness on the floor apron 122, to build up several thicknesses of criss-crossed web to form a batt 124 of, for instance, about 90 to 120 inches (2.3 to 3.0 m) in width and about 4 inches (10 cm) in thickness. During carding, the material is stretched and pulled into a cloth-like mat consisting primarily of parallel fibers. With nearly all of its fibers extending in the carding direction, the mat has some strength when pulled in the carding direction but almost no strength when pulled in the carding cross direction, as cross direction strength results only from a few entanglements between fibers. It is important to note that the carding direction is not the machine direction of the finished product. During crosslapping, the carded fiber mat is laid in an overlapping zigzag pattern, creating batt 124 of multiple layers of alternating diagonal fibers. The diagonal layers, which extend in the carding cross direction, extend more across apron 122 than they extend along its length. For instance, we have used batt which has been crosslapped to form layers extending at anywhere from about 6 to 18 degrees from the cross direction of the finished product. The resultant crosslapped batt 124, therefore, has more cross direction strength (i.e., across apron 122) than it has machine direction strength (i.e., along apron 122). Note that the machine direction of the final product is in the same sense as the direction along apron 122. Batt 124 has little machine direction strength because the fiber layers are merely laid upon one another and are in no way woven together. The material properties and the manufacturing process can be affected by the crosslapping angle. A steeper angle may balance the cross and machine direction strengths, which may affect fastener performance and the ease of manufacturing. With more machine-directional crosslapping, in some cases the initial machine direction stretch described below may be eliminated while still obtaining a useful product.

In preparation for needling, batt 124 is gradually compressed in a tapered nip between floor apron 122 and a moving overhead apron 138 to reduce its thickness to about one inch. A relatively thin, low density batt can thus be produced.

Needling of batt 124 is performed in multiple, sequential needling stages in order to provide a very high density of needle penetrations without destroying the low density batt. In the presently preferred method felting needles are employed having fiber-engaging barbs on their sides. Needle punching gives the batt cohesion. The needle barbs pull fibers from one layer of the batt through other layers, entangling the fibers from different layers that are oriented in different directions. The resulting entanglements hold the batt together.

From floor apron 122, the batt is passed to a first needle loom 140 with two needling stations 142 and 144 having rows of notched (i.e., barbed) needles. Needling station 142 needles the batt of staple fibers from its upper surface at a density in the range of 100 to 160 punches per square inch (15 to 25 per square cm). In this embodiment, the batt was needled at a density of 134 punches per square inch (21 per square cm). Subsequently, needling station 144 needles the once-needled batt a second time, with needles which penetrate the batt from its upper surface at a density in the range of 500 to 900 punches per square inch (78 to 140 per square cm) to produce a needled batt 146. In this example, the second needling is at a density of 716 punches per square inch (111 per square cm). We refer to the operation of loom 140 as the first needling stage. Additional information on needling processes can be obtained from the Association of the Nonwoven Fabrics Industry (INDA) of Cary, North Carolina, which publishes the INDA Nonwovens Handbook.

After the first needling stage, needled batt 146 is passed between drive rolls 148 and into a J-box accumulator 150 which, besides holding a bank of batt to accommodate variations in processing rates, allows the needled batt to relax and cool before entering the second needling stage. Alternatively, the needled batt 146 may be spooled after the first needling stage, with subsequent operations performed on a second line. If materials and conditions allow, the needled batt may be passed directly from the first needling stage to the second needling stage without accumulation, but care should be taken to ensure that the batt is sufficiently cool and relaxed to withstand the second needling stage.

As a result of the needle punching, the fibers of the batt become highly randomized and chaotic. However, the underlying pattern of alternating diagonals remains unchanged, although obscured.

From J-box accumulator 150, needled batt 146 is pulled through a guider/spreader 152 (of, e.g., the one-over-two configuration) to properly apply light tension to the batt as is customary for needling, without significant stretching of the batt. It then passes through a second needle loom 154 for a second needling stage. The operation of this second stage is referred to "super needling", as it is a very dense secondary needling operation and produces many loops of substantial loft. Loom 154 has a single needling station 156 in which needled batt 146 is needled from the lower side to produce high-loft loops extending from the upper side. To produce such loops, the sharp tips of the notched needles of loom 154 are extended a substantial distance (e.g., about 1/4 inch or 6.3 mm) beyond the thickness of the batt in the opposite direction as the needles of the first needling station, pushing individual fibers away from the bulk of the batt to form upstanding loops. When the needles retract, the loops remain. The loops may be formed of fibers which originally lay on the opposite side of the batt, or from fibers drawn from the middle of the batt. In either case, the needles drag fibers out of the batt and leave them extending from the bulk of the batt as loops which give one side of the super-needled batt a fuzzy appearance.

This super-needling process does not require special needling bedplates or supporting brushes into which the needles extend, such as are employed in structured or random velour looms, although such techniques may be employed to advantage, e.g., where large loops are desired for use with large hooks. The super-needling is primarily characterized as an extremely dense needling, on the order of about 1000 to 2000 punches per square inch (155 to 310 per square cm), or preferably about 1400 punches per square inch (217 per square cm). Standard barbed needles are employed, such as triangular section 15x18x42x3 C222 G3017 felting needles from Groz-Beckert. During this secondary needling operation, individual fibers of the batt are pushed through the loop side of the batt to produce loose, relatively lofty loops. Together, these loops give the loop side of the super-needled batt a fuzzy appearance and feel. Too much extension of the individual loop fibers at this point can cause them to break during subsequent stretching, so the distance the needles extend through the batt is selected in consideration of the denier and tenacity of the fibers used. We have found that extending the needles about 1/4 inch (6.3 mm) beyond the batt works well for 6 denier fibers with a tenacity of about 3.5 grams per denier.

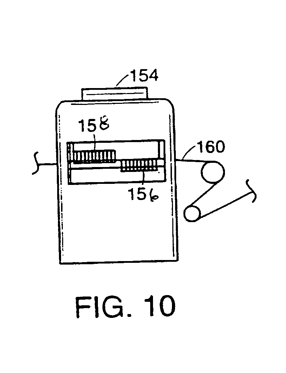

In one embodiment, illustrated in Fig. 10, needle loom 154 has an additional, second needling station 158. After producing high-loft loops extending from one surface, the batt is super needled in the other direction to produce loops extending from its other surface, such that both sides have extended loops.

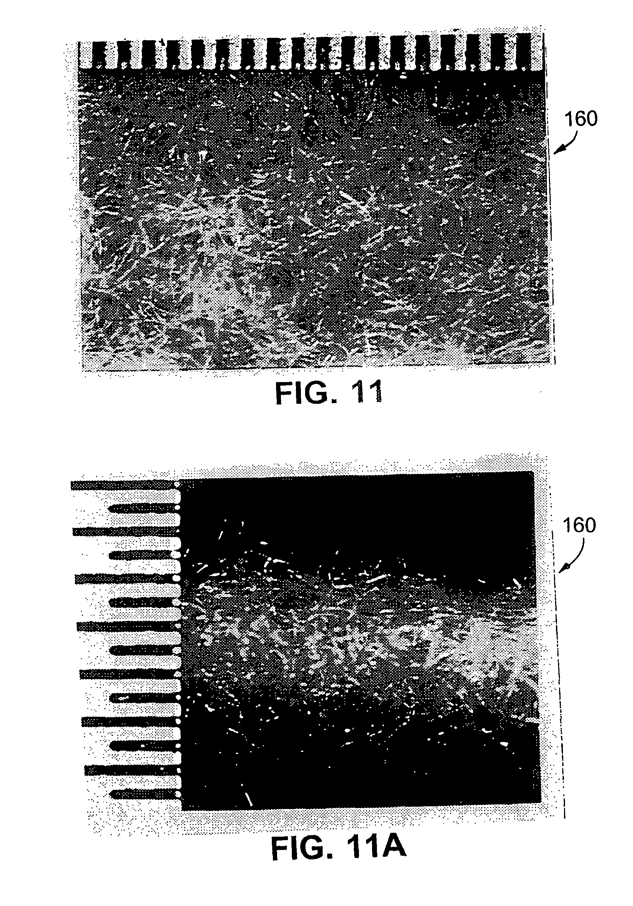

After leaving loom 154, super-needled batt 160 is split into two running 45 inch (114 cm) widths and spooled on rolls 162. As shown in Fig. 11, the fibers of batt 160 have been entangled by the needle punching process to create loose entanglements throughout the batt. At this stage, the batt is not an acceptable loop product for many hook-and-loop fastening applications, as the individual loops may be relatively easily pulled away from the batt and are not well anchored at the entanglements.

After super-needling, the loop definition, see Fig. 11A, on the working side of the batt is also not as distinct as it can be after the stretching that is employed to produce products with loop trees. This structural difference can be seen by comparing Figs 11, 11A with Figs. 2 and 3, for instance.

In another embodiment (not illustrated), the second needling stage is omitted. Instead, needle looms 142 and 144 of the first needling stage (Fig. 9) are configured to super-needle the batt in both directions. Loom 142 needles the batt from the top at a rate of 254 punches per square inch (39 per square cm), with the needles penetrating the batt and extending through the bottom of the batt a distance of 10.2 millimeters. Loom 144 then needles the batt from the bottom at a rate of 254 punches per square inch (39 per square cm), with the needles penetrating the batt and extending through the top of the batt a distance of 7.1 millimeters to form loops on the top side of the batt. The needles of loom 144 tend to take fibers that have been pushed through the bottom of the batt by the needles of loom 142 and force them back up through the batt in the formation of topside loops. Although this process results in a relatively small number of loops on the bottom of the finished product, due to the first needling of loom 142, the resulting product has been found to be useful for some applications. The needling density, speed, and penetration of looms 142 and 144 may be varied to produce a product with substantially no backside loops, or with hook-engageable loops extending from both sides.

The batt following super needling has a fair amount of loft and resiliency, with the loops and other fibers of the batt forming loose, gentle arches between entanglements. At this point the batt is very flexible, and the density of fibers gradually decreases away from either side of the material. At first glance, it can be difficult to tell which side has been super-needled, if only one side has been subjected to that action. Batt 160, in this example, has an overall thickness, including loops, of about 3/16 inch (4.8 mm) and a weight of between about 2 and 4 ounces per square yard (68 and 135 grams per square meter).

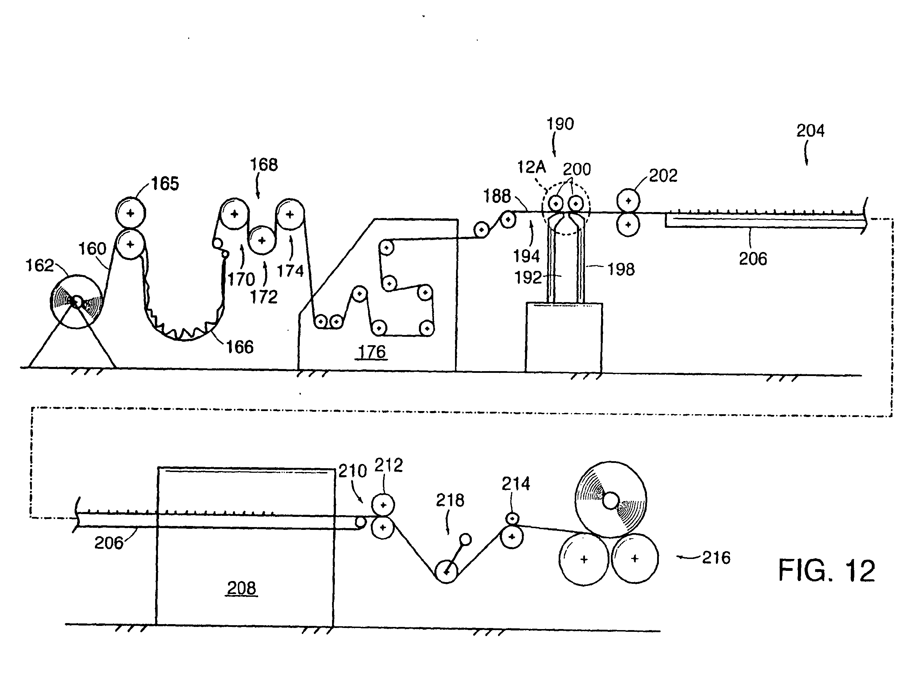

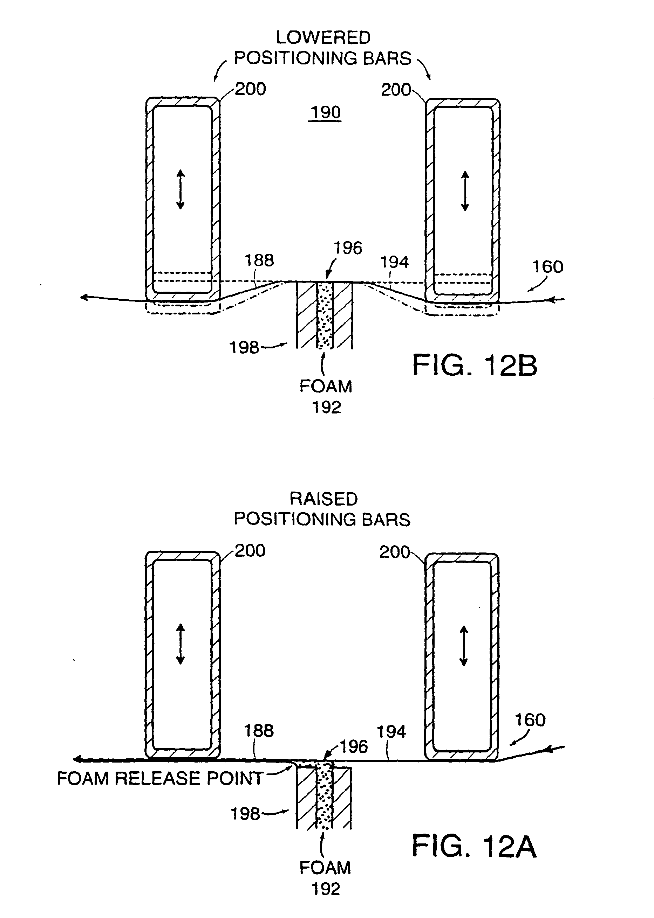

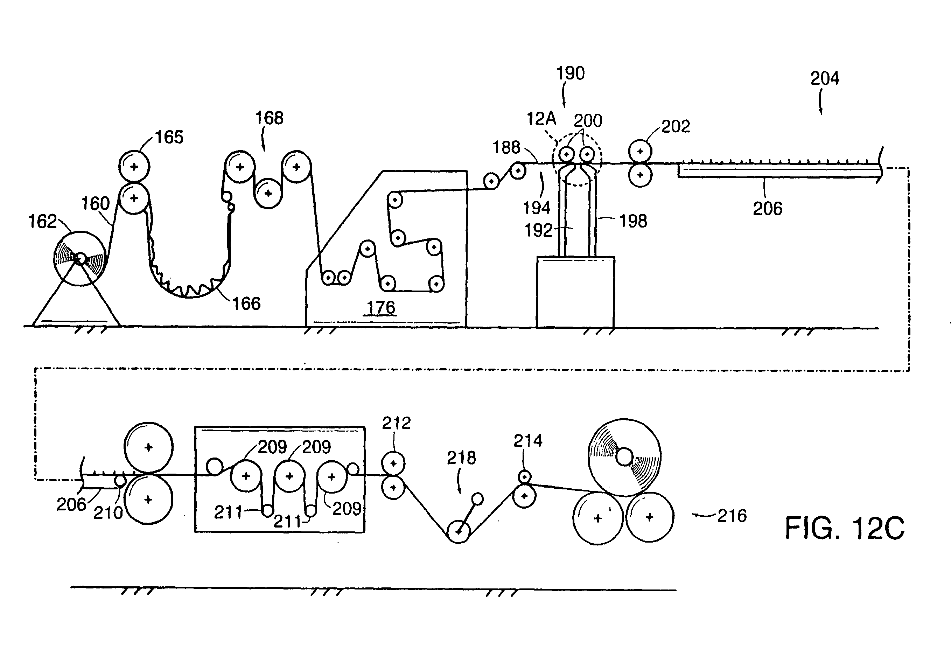

Referring to Fig. 12, a spooled length of super-needled batt 160 is spooled from roll 162 by drive rolls 165 and into a J-box accumulator 166, allowing roll 162 to be replaced and the batt spliced without interrupting further processes. The J-box also allows the batt to recover from any elastic deformation caused by the spooling process. Batt 160 is pulled from accumulator 166 through a guider 168 to center the batt in the cross-machine direction. Guider 168 includes three rolls in a two-over-one configuration. The first and second rolls 170 and 172 have left and right herringbone pattern scroll surfaces originating at the center of the roll that, being slightly overdriven, urge any wrinkles in the batt toward its edges to remove them. The third roll, roll 174, is a split braking roll to controllably tension either half of the batt to guide the fabric to the left or right as desired.

From guider 168 the batt passes through a tension controller 176 that maintains a desired tension in the batt through the subsequent binder application process. Controlling the difference between the speed of tension controller 176 and downstream drive rolls 202 applies a desired amount of machine direction stretch to the batt prior to cross-machine stretching. In some cases, no substantial machine direction stretch is purposefully applied, any noted machine direction lengthening being due only to minimal web processing tension in the supply batt. In other cases, machine direction stretch is purposefully induced by running drive rolls 202 faster than tension controller 176.

In the embodiment in which batt 160 has been super-needled to produce loops extending from only its front side 188, batt 160 is next passed through a coating station 190 in which a foamed, water-based adhesive 192 (i.e., a water-based adhesive, whipped to entrain air) is applied to the back side 194 of the batt across its width.

Referring also to Figs. 12A and 12B, the foamed liquid adhesive is pumped at a controlled rate through a long, narrow aperture 196 in the upper, surface of the applicator 198 as the batt is wiped across the aperture, thereby causing the adhesive to partially penetrate the thickness of the batt. Positioning bars 200, on either side of aperture 196, are raised and lowered to control the amount of pressure between batt 160 and applicator 198. The depth of penetration of the adhesive into the batt is controlled (e.g., by the flow rate and consistency of adhesive 192, the speed of batt 160 and the position of bars 200) to sufficiently coat or penetrate enough of the fiber entanglements to hold the product in its final form, while avoiding the application of adhesive 192 to the loop-forming fiber portions of the front side 188 of the batt. The foaming of the liquid adhesive before application helps to produce an even coating of the back side of the batt and helps to limit penetration of the fluid adhesive into the batt. After the semi-stable foam is applied it has a consistency similar to heavy cream, but the bubbles quickly burst to leave a liquid coating that flows as a result of wetting and surface tension, into the tightening fiber entanglements. Alternatively, the foam may have a thicker consistency, more like shaving cream, to further reduce the penetration into the batt and form more of a distinct resinous backing. A non-collapsible (i.e., stable) foam of urethane or acrylic, for instance, is useful to produce a radio frequency-weldable backing which functions as a water barrier. Such a product has particular application to disposable garments and diapers.

It is important that the binder (e.g., adhesive 192) not interfere with the loop-forming portions of the fibers on the front side 188 of the batt. It is not necessary that the knot bases be completely covered by binder; it is sufficient that they be secured by the binder in the finished product to stabilize the fabric against significant further stretching and to strengthen the bases of the loops. Preferably, the binder is at least partially in liquid form to wick into the entanglements before and while they are subsequently tightened during stretching. The capillary action of the liquid binder is such that examination of the finished product shows that the binder is almost exclusively at the knots of the web (at the base of the loops, for instance), and therefore does not tend to adversely affect either the functionality of the free-standing loops or the flexibility of the web.

After leaving coating station 190, the material is subjected to stretching in the plane of the web. In the presently preferred case the web is wound through variable speed drive rolls 202 and onto a tenter frame 204 for cross-machine stretching (i.e., stretching in the cross-machine direction). The speed of drive rolls 202 is adjustable, with respect to both tension control 176 and the rails 206 of the tenter frame, to cause a predetermined amount of machine direction stretch in the batt, either between tension control 176 and drive rolls 202, or between drive rolls 202 and frame rails 206, or both. In some embodiments no permanent machine direction stretch is applied, but the batt is nevertheless held in tension to control adhesive penetration and maintain proper frame rail pin spacing. In other embodiments the batt is generally stretched, in total, between about 20 percent and 50 percent in the machine direction before tentering.

As it enters tenter frame 204, the 45 inch wide batt 160 is engaged along its edges by pins of frame rails 206 that maintain the machine-direction dimension of the material as it is stretched in the cross-machine direction. The spacing (of, e.g., about 3/16 inch or 4.8 mm) between adjacent pins is maintained throughout the length of the tenter frame, such that no additional machine-direction stretch is applied. Due to the needling, batt 160 should have enough tensile strength to be properly engaged by the rail pins and withstand the subsequent cross-machine stretching.

Tenter frame 204 has a tapered section where the rails 206 separate at a constant, adjustable range rate over a machine-direction length of about 10 feet (3 meters) to a final width which can range from 45 inches (114 cm) to about 65 to 69 inches (165 to 175 cm). This equates to a cross-machine stretch, in this particular embodiment, of about 50 percent. In general, to take advantage of the economics that can be realized according to the invention, the batt should be stretched to increase its area by at least about 20 percent (we call this "percent areal stretch"), preferably more than about 60 percent areal stretch and more preferably more than about 100 percent areal stretch, to increase the area of the product while tightening the binder-containing entanglements of the batt that contribute to improvement in the strength of anchorage of the individual loops. We have found that in some cases the super-needled batt can be stretched, by employing the above method, at least 130 areal percent or more and provide very useful hook-engaging properties. The more the stretch, the greater the overall yield and the lighter in weight the final product. Even greater overall cross-machine stretch percentages can be employed, for instance by using multiple tentering stages in situations wherein the batt is constructed to withstand the stretch and still be able to reasonably engage hooks. In one instance, the super-needled batt described above was stretched from an initial width of 45 inches (114 cm) to 65 inches (165 cm), softened (by adding a softener), slit to a 45 inch (114 cm) width, stretched a second time to a 65 inch (165 cm) width before applying a binder, and still had useful, hook-engageable loops. In some cases final product widths of 6 to 8 feet (1.8 to 2.4 meters) or even much more can be achieved.

In a preferred embodiment, the non-woven web starting material used to manufacture the loop component is a fairly dense, needle punched, non-woven web of fibers lying in an apparently chaotic and tangled manner. One side, the "fuzzy side", has an excess of large, loose loop fibers created during a second needle punching process. The web is first stretched to 130 percent of its initial length in the machine direction. This stretching results in necking -- the material narrows to 80 percent of its initial width, from 45 inches (114 cm) to 36 inches (91 cm). It is then coated with a binder. Next, it is stretched to 175% of its necked width, from 36 inches (91 cm) to 63 inches (160 cm). During this process the material becomes much more sparse, with spider-like clusters of fiber (see bases B of Fig. 2B) serving to anchor the loop. The mechanism by which this change occurs relates to the method by which initial/non-woven web is manufactured.

When loop material formed of a highly diagonal crosslapped batt is stretched in the machine direction, it is observed that little tension is placed upon the constituent fibers. This is because the vast majority of the fibers run in a diagonal direction that lies close to the cross machine direction. Applying machine direction tension tends to increase the angles at which the diagonals lie (i.e., move them toward a 45 degree angle with the machine direction). They become steeper, or are turned to a degree toward the machine direction, much like the angles of the legs on a folding chair grow steeper when the chair is opened. And just as the leg base of a folding chair gets narrower as the chair gets taller, so does the material neck and lose width as it is stretched. This is evidenced by breaking a piece of such material in the machine direction: the fibers do not break, they merely pull apart from each other. Because little tension is put on the fibers until they are nearly parallel, a mere 30 percent stretch does not disturb the chaotic arrangement of the fibers and little change can be seen under the microscope. Even though the fibers are re-orienting, the arrangement looks no less chaotic, since the fibers themselves are never brought under enough tension to straighten them out.

The second stretch of the above embodiment, performed in the cross machine direction, produces drastic changes. Though somewhat more angled as a result of the first stretch, the fibers still extend more in the cross machine direction than in the machine direction. The fact that the fibers are oriented closer to cross machine direction means that a smaller elongation is required before the fibers are close enough to the cross direction to experience tension. Tension, when applied, causes the fibers to try to straighten themselves out and re-orient into the cross machine direction. If the web were not previously needle punched, the material would probably lose all cohesion at this point. However, the needle punching of the web causes fibers from different layers with different orientations to entangle one another. Because of these entanglements, the fibers cannot straighten. As cross machine tension is applied the entanglements tend to bunch together to generate knots that resemble "spiders" as they have a core with many taught legs emanating from the core in different directions. Each spider forms at a point where a needle caused an entanglement of multiple fibers, which tried to pull apart during the cross direction stretch. The bunching of the entanglements gives them the spider like appearance. Each loop of the fuzzy side corresponds to a point where a needle was punched through; consequently, after the cross direction stretch each fuzzy side loop lies at the center of a corresponding spider-like knot. This was seen in one experiment where the functional loops (i.e., the outer region of the fuzzy side) of the product were colored purple. All of the purple in the colored product was visible at the centers of the spiders formed at the bases of the loops. On the other hand, when the non-fuzzy side of the fabric was colored, no gathering of color at the center of the spiders was observed. Only the fuzzy side, functional loops corresponded to needle punches, and only these loops were observed to have spiders form around them. It is believed that the presence of the loop and its corresponding entanglement is largely responsible for the formation of the spider or knot. The fact that a loop is pulled through the web means that there is now a vertical fiber (i.e., a fiber extending out of the plane of the web) around which the horizontal fibers of the web entangle. Thus most loops in the finished loop tape have a spider at their base, which provides increased strength for anchoring the loop.

Stretching the experimental, purple-looped sample past the point at which the loops are of maximum height tended to draw the purple tinted loops back into the plane of the web. The greater the stretching, the smaller the loops grew, until finally they began to pull out of their entanglements. As this happened, the spider that had formed around them disappeared and the entangled fibers straightened and sank back into the web. With increased stretching, after many loops are drawn back and their entanglements vanish, the material loses its cohesion, the fibers slide past one another, and the material parts.

From this examination, it appeared that the material is stronger in the cross machine direction, attributed to the fact that the carded fabric is crosslapped at an angle closer to this direction. By changing the carding angle progressively away from the cross-machine direction, more strength in the machine direction should be achievable. Also, machine direction stretching tends to re-orient the fibers towards the machine direction. However, since they begin close to cross direction, the mere 30 percent elongation that the material undergoes in the above-described longitudinal stretching is insufficient to place enough tension on the fibers to straighten them or to cause spiders or knots to form. However, machine direction stretching is considered important for highly cross-directional crosslapped batts, which would otherwise have a lay which is too cross-directional for a uniform cross direction stretch to be achieved; in that case the fibers are so close to cross-directional that they do not entangle as significantly, nor are they properly spaced in the final product. Instead, they remain very near to each other and nearly parallel. When the spiders do form in such instances, they are elongated in the cross direction and very close together, and the end material is much more dense.

Because in the above-described embodiment the fibers are already more cross-directional than machine-directional, and because the cross direction stretch applied is greater, the fibers are placed under tension during cross direction elongation. Fibers entangled during needle punching tend to clump together, and as the fibers tend to straighten, these entanglements form spider-like radial patterns of fibers.

It was also noted that spiders form at the location of functional loops created by deep needle punching, the fibers of which have been drawn through some or all of the web. Consequently, the loop fibers entangle the other fibers and form spiders. This means that in the finished product, the loops have spiders at their base, locking the loop fibers into the web.

Another way of saying this is that the "loop trees" (see Fig. 3B), which do not distinctly appear in the pre-stretched batt, obtain their final form as fibers of the ground portion of the web are pulled and the entanglements beneath them are tightened during stretching to form knots. As the batt is stretched, the tension in the taut fibers of the web forces some of the loop trees to stand erect, such that the overall thickness of the stretched batt (with functional loops) can actually be greater than the unstretched batt. To extend the horticultural analogy, the homogeneous thicket of the loop surface of the unstretched batt becomes the orchard of spaced clusters of the stretched product. Although the loop trees or loop formations correspond to locations where the batt was punched during super-needling, the resulting "orchard" of loop formations does not exhibit the same ordered pattern, after the web is stretched, as might be anticipated by the pattern of punches of the needling process. We believe that the arrangement of loop formations is randomized during the stretching process, as distances between entanglements change as a function of the properties, direction and number of the fibers connecting various nodes. The resulting product has no apparent order to the arrangement of loops extending from its surface.

Despite the relatively wide loop spacing that is achieved, the loops, after curing of the binder, are found to be so strongly anchored and so available for engagement by the hooks, that a web unusually treated according to these techniques can perform in an excellent manner despite having a gossamer appearance.

Referring back to Fig. 12, while the stretched batt is held on frame rails 206 in its stretched condition it is passed through an oven 208 in which the product is heated to dry and cross-link acrylic binder 192 and stabilize the dimensional integrity of the batt. Oven 208 is essentially a convection drier with air venturi nozzles which blow hot air up into and down onto the web to evaporate some of the water of the adhesive. In this example, the heating time and temperature are about one minute and 375 degrees F (190 degrees C), respectively. In some embodiments (not shown), the batt is retained on frame rails 206 for secondary coating passes through additional coating stations and drying ovens, thereby building up a desired laminate structure for particular applications.

In another embodiment, hook-engageable loops are formed on both sides of the web by needling, by the super-needling techniques acting on staple fibers that have been described, or by other known techniques. After forming the loops the web is passed through a bath of binder. In some cases, where the loops are relatively stiff and the binder is of suitably low viscosity, after removal from the bath the binder drains from the loops and the loops, by their own resiliency and stiffness resume their free-standing stance while capillary action retain the binder in the center of the fabric. The web is then subjected to stretching and curing as above.

In other instances, as where the loop material is less stiff, auxiliary means are employed to remove excess binder after passing through the bath, as by passing the fabric through a nip of squeeze rolls, or by subjecting both sides of the fabric to an air knife, or by blotting followed, in each case for instance by blowing air or otherwise loosening the loops and causing them to stand upright.

Other embodiments carrying hook-engageable loops on one or both sides, and incorporating heat-fusible binder fibers or other heat-fusible binding constituents, are bonded by non contact means such as by blasts of hot air directed at both sides of the fabric at temperatures sufficient to melt the heat-fusible binding material and lock the fabric structure in its stretched condition.

Heat fusible fibers or other material colored black or otherwise adapted to absorb radiant heat, may be activated by radiant heaters to bind the ground portion of the fabric following stretching. Care must be taken, in such instances, to avoid mitigation of the engagement properties of the free-standing loops.

Referring back to Fig. 12, the stretched batt exits oven 208 still attached to the pins, and is then pulled from the pins by a de-pinning device 210 and a pair of drive rolls 212. The finished, wide batt is then slit, if desired, into appropriate multiple widths by a slitter 214 and spooled on a driven surface winder 216. A dancer 218 between drive rolls 212 and slitter 214 monitors the tension in the batt to control the speed of winder 216. Slitter 214 can also be used to trim off the edges of the batt that include the material outboard of the frame rails through the tenter frame. Optionally, the finished batt can be brushed before or after spooling to disentangle loosely-held loop fibers to improve the consistency of the closure performance between the first and subsequent engagements with a hook product.

Referring to Fig. 12C, an alternative process employs heated rolls or "hot cans" 209 or platens to stabilize the back side of the fabric in its stretched condition. This embodiment does not require a coating or adhesive when using thermoplastic fibers, as the fibers are locally fused together by heat. Cooled rolls 211 engage the loop side of the fabric during passage, to prevent damage to the hook-engageable loops.

In order to produce the product of Fig. 6, the longitudinal continuous fiber strands 28 are added to the batt prior to needle punching. These fibers are preferably crimped enough to allow the fibers to retain their integrity after the needle-punching process, to be stretched a limited amount in the machine direction as the batt is stretched.

It will be understood that the above-described stretching technique can be employed to advantage on other stretchable, loop-defining non-woven webs. Thus, in its broadest aspects, the invention is not to be limited to the use of needled webs. Webs formed by hydro or air current entanglement can, for instance, be employed.

In most cases where significant strength performance is desired, it is preferable to employ non-woven materials formed of staple fibers to take advantage of their drawn, molecular oriented structure, or other fibers of the substantial tenacity.

Other features and advantages of the invention will be realized, and are within the scope of the following claims.

## Claims

1. A loop fastener component of a hook-and-loop fastener comprising a non-woven web (12) of entangled fibers, having a generally planar web body (16) from at least one face of which fibers in the form of hook-engageable loops (14) extend, **characterized in that**
the web (12), including the web body (16) and the loops (14), has weight less than about 4 ounces per square yard (135 grams per square meter), that
the web body comprises a non-uniform distribution of fibers in which fibers are in relatively high concentrations in regions of bases (B) of the hook-engageable loops (14), and in relatively lower concentrations in regions (R) lying between the bases (B) of the loops, and that
a substantial number of fibers in regions (R) of lower concentration are taut in the plane of the web body (16), and extend in different directions radiating from the bases (B) of the loops (14).

2. The loop fastener component of claim 1 **characterized in that** the bases (B) of the loops (14) contain taut portions of fibers of the web body trained about loop-forming fibers.

3. The loop fastener component of claim 2 **characterized in that** taut fibers of the bases (B) contribute to the definition of free-standing formations (20) that extend from the plane of the web body (16) and contain the hook-engageable loops (14).

4. The loop fastener component of claim 3 **characterized in that** the free-standing formations (20) comprise elongated trunks (22) from each of which multiple hook-engageable loops (14) extend.

5. The loop fastener component of any of the preceding claims **characterized in that** the relatively high concentrations of fibers at the bases (B) of hook-engageable loops define tightened fiber entanglements.

6. The loop fastener component of any of the preceding claims **characterized in that** the combined weight of the web body (16) and the loops (14) is less that about 2 ounces per square yard (68 grams per square meter).

7. The loop fastener component of any of the preceding claims **characterized in that** the loops (14) are disposed across the face of the web body (16) in a relatively random pattern.

8. The loop fastener component of any of the preceding claims **characterized in that** a solidified fluid binder (21) is concentrated at the bases (B) of the loops.

9. The loop fastener component of claim 8 **characterized in that** the solidified fluid binder (21) is selected from the group consisting of acrylics, urethanes, polyvinyls, formaldehydes, glyoxals and epoxies.

10. The loop fastener component of claim 8 or 9 **characterized in that** the solidified fluid binder (21) is configured at the bases (B) of the loops at least partially as a result of capillary flow prior to solidification.

11. The loop fastener component of any of claims 8 through 10 **characterized in that** the solidified fluid binder (21) composes between about 20 and 40 percent of the total weight, preferably about one third of the total weight, of the web (12), including the loops (14).

12. The loop fastener component of any of claims 1 through 7 combined with a layer (302) of thermoplastic material, a second face of the web body (16), opposite the face from which hook-engageable loops (14) extend, being encapsulated in the layer of thermoplastic material.

13. The loop fastener component of claim 12 **characterized in that** hook elements (304) are integrally molded on a surface of the layer (302) of thermoplastic material.

14. The loop fastener component of any of the preceding claims **characterized in that** thermoplastic fibers of the web (12) are heat-fused at the bases (B) of hook-engageable loops (14).

15. The loop fastener component of any of the preceding claims formed from a needled non-woven starting batt (160) of given dimension, **characterized in that** the web (12) of the loop fastener component, in its general plane of extent, is in a stabilized stretched condition of at least 20 percent greater area, preferably at least 50 percent greater area, and more preferably at least 100 percent greater area, than the area of the starting batt (160).

16. The loop fastener component of claim 15 **characterized in that** the web (12) is in a stretched condition of at least 20 percent in at least a first direction in the general plane of extent of the web (12).

17. The loop fastener component of claim 15 **characterized in that** the web (12) is in a stretched condition of at least 20 percent in each of two orthogonal directions in the general plane of extent of the web.

18. The loop fastener component of any of claims 15 through 17 **characterized in that** the starting batt (160) comprises layers of generally parallel fibers with fibers of some layers oriented at acute angles with respect to fibers of other layers, **characterized in that**, as a result of stretching, the acute angles between fibers of the web body (16) corresponding to different layers of the starting batt (160) are generally greater than corresponding angles in the starting batt (160), while at least many of the fibers corresponding to each layer remain relatively parallel.

19. The loop fastener component of claim 18 **characterized in that** the layers of a longitudinally extending starting batt (160) extend predominantly transversely across the batt, **characterized in that**, as a result of longitudinal stretching of the starting batt (160), the acute angles between fibers of the web body (16) corresponding to different layers of the starting batt (160) are generally greater than corresponding angles in the starting batt (160).

20. The loop fastener component of any of the preceding claims having an overall thickness (t), including the web body (16) and a majority of the loops (14), of less than about 0.150 inch (4.0 mm), preferably less than about 0.100 inch (2.5 mm).

21. The loop fastener component of any of the preceding claims **characterized in that** the hook-engageable loops (14) extend from associated loop bases (B) within the plane of the web to an average loop height (hL), measured as the perpendicular distance from the web body (16), of between about 0.020 and 0.060 inch (0.5 and 1.0 mm).

22. The loop fastener component of any of the preceding claims **characterized in that** the web (12) has an overall thickness (t), including the web body (16) and a majority of the loops (14), and wherein the average loop height (hL) is between about 0.5 and 0.8 times the overall thickness (t) of the product.

23. The loop fastener component of any of the preceding claims having between about 50 and 1000 tightened fiber entanglements per square inch (8 and 160 per square cm) of web area, from which hook-engageable loops (14) extend.

24. The loop fastener component of any of the preceding claims **characterized in that** the web (12) is generally composed of fibers having a tenacity of at least 2.8 grams per denier, preferably at least 5.0 grams per denier, and more preferably at least 8.0 grams per denier.

25. The loop fastener component of any of the preceding claims **characterized in that** the loops (14) extend from the web body (16) to varied heights (h_{L}) to form a multi-level arrangement of hook-engageable loops (14).

26. The loop fastener component of any of the preceding claims having a Gurley stiffness of less than about 300 milligrams.

27. The loop fastener component of any of the preceding claims including separated strengthening strands (28) extending at least in one direction within the plane of the web body (16).

28. An enclosure (56) comprising a first closure portion (60) having loops, and a second closure portion (58) having hooks constructed to engage the loops of the first closure portion to hold the enclosure (56) in an open or closed position, the enclosure **characterized in that** the first closure portion (60) comprises the loop fastener product of any of the preceding claims.

29. The enclosure of claim 28 **characterized in that** the first and second closure portions (58 and 60) have a combined overall thickness, engaged and at rest, of less than about 0.075 inch (1.9 mm).

30. A disposable article of clothing (50, 54) having a fabric and a fastener with hooking elements arranged to engage loops of the fabric to form a releasable fastening to retain the article of clothing (50, 54) on a wearer, the article of clothing **characterized in that** the fabric comprises the loop fastener component of any of claims 1 through 27.

31. An air filter (80) **characterized in that** the filter comprises the loop fastener product of any of the claims 1 through 27 arranged to intercept and filter a flow of air.

32. A geotextile barrier (352) **characterized in that** the barrier comprises the loop fastener product of any of the claims 1 through 27 arranged to intercept and filter a flow of groundwater.

33. A geotextile barrier (352) **characterized in that** the barrier comprises the loop fastener product of any of the claims 1 through 27 arranged to stabilize a soil layer boundary.

34. A fastener product having a sheet-form polymer base (302) with hooks (304) integrally molded with and extending from one side of the base, and a loop material permanently bonded to and extending over an opposite side of the base, the product **characterized in that** the loop material of the product comprises the loop fastener material (12) of any of claims 1 through 27; and that the loops (14) of the loop fastener material (12) are adapted to be engaged by the hooks (304) of the product.

35. In combination,
a hook fastener product (10) having hooks extending from a surface thereof; and
the loop fastener product of any of claims 1 through 27, the hooks of the hook fastener product being adapted to engage the loops (14) of the loop fastener product to form a releasable fastening.

36. The combination of claim 35 **characterized in that** the hook fastener product (10) and the loop fastener product have a combined overall thickness, engaged and at rest, of less than about 0.075 inch (1.9 mm), preferably less than about 0.050 inch (1,27 mm).

37. A method of forming a loop fastener component for hook-and-loop fastening from a generally planar non-woven batt (160) of entangled fibers, the method **characterized by**
stretching the batt (160) in at least one direction, thereby producing a stretched web (12) having a generally planar web body (16) with a non-uniform distribution of fibers, with fibers in relatively high concentrations in regions of bases (B) of hook-engageable loops (14) extending from the web body (16), and in relatively lower concentrations in regions (R) of the web between bases (B) of the loops, with
a substantial number of fibers in regions (R) of lower concentration being taut in the plane of the web body (16), and extending in different directions radiating from the bases (B) of the loops; and
stabilizing the web (12) in its stretched condition.

38. The method of claim 37 **characterized in that** the web (12) is stabilized in its stretched condition by solidifying a fluid binder (21), applied to the web body (16) under conditions that concentrate the binder at the bases (B) of the loops.

39. The method of claim 37 **characterized in that** the web (12) is of weight less than about 4 ounces per square yard (135 grams per square meter).

40. The method of claim 39 **characterized in that** the stretching causes taut fibers of the web body to be trained about loop-forming fibers in the bases (B) of the loops (14).

41. The method of any of the preceding method claims **characterized in that** the batt is stretched in a manner that the relatively high concentrations of fibers at the bases (B) of hook-engageable loops define tightened fiber entanglements.

42. The method of any of the preceding method claims **characterized in that** the batt is stretched so as to produce a web (12) having a weight, including the loops (14), of less than about 2 ounces per square yard (68 grams per square meter).

43. The method of any of the preceding method claims **characterized in that** the web (12) is stabilized in its stretched condition by solidifying a fluid binder (21), applied to the web body (16) under conditions that concentrate the binder at the bases (B) of the loops.

44. The method of claim 43 **characterized in that** the fluid binder (21) is applied prior to at least some of the stretching.

45. The method of claim 43 **characterized in that** a selected amount of binder (21) is applied, such that the binder composes between about 20 and 40 percent of the total weight, preferably about one third of the total weight, of the web (12), including the loops (14).

46. The method of any of the preceding method claims **characterized in that** the batt (160) is provided with fusible thermoplastic fibers embedded therein, and that the stretched web is stabilized under conditions which cause the fusible thermoplastic fibers to become heat-fused at the bases (B) of hook-engageable loops.

47. The method of any of the preceding method claims **characterized in that** the web (12) is stabilized in a stretched condition in which the web has, in its general plane of extent, an area at least 20 percent greater, preferably at least 50 percent greater, more preferably at least 100 percent greater, than the area of the batt (160) prior to stretching.

48. The method of claim 47 **characterized in that** the batt (160) is stretched at least 20 percent in a first direction in the general plane of extent of the web body (16).

49. The method of claim 47 **characterized in that** the batt (160) is stretched at least 20 percent in each of two generally orthogonal directions in the general plane of extent of the web body (16).

50. A method of filtering air, **characterized by** arranging the web (12) of the loop fastener product of any of claims 1 through 27 to intercept a flow of air to be filtered.

51. A method of filtering groundwater, **characterized by** arranging the web (12) of the loop fastener product of any of claims 1 through 27 to intercept a flow of groundwater to be filtered, and by securing the web in position by engaging the loops (14) of the loop fastener product with a length of hook fastening tape (360).

52. A method of stabilizing soil, **characterized by** arranging the web (12) of the loop fastener product of any of claims 1 through 27 between adjacent layers of soil to be stabilized, and by securing the web in position by engaging the loops (14) of the loop fastener product with a length of hook fastening tape (360).

## Patentansprüche

1. Schlingen bzw. Schlaufen aufweisende Befestigungskomponente eines Klettverschlusses, umfassend eine nichtgewebte Fasermatte bzw. -bahn bzw. ein nichtgewebtes Vlies (12) aus verwickelten bzw. verknäuelten Fasern mit einem im Allgemeinen ebenen Fasermatten- bzw. Vlieskörper (16) von dem sich mindestens von einer Fläche Fasern in Form von einhakbaren bzw. Haken ergreifenden Schlingen (14) bzw. Schlaufen erstrecken, **dadurch gekennzeichnet, dass**
die Fasermatte bzw. das Vlies (12), einschließlich des Fasermatten- bzw. Vlieskörpers (16), ein Gewicht von weniger als 4 Unzen/Quadratyard (135g/m²) aufweist,
der Fasermatten- bzw. Vlieskörper eine ungleichmäßige Faserverteilung umfasst, in der die Fasern in den Regionen der Basis (B) der einhakbaren Schlingen (14) in relativ hoher Konzentration und in Regionen (R), die zwischen den Basen (B) der Schlingen liegen, in relativ geringer Konzentration vorhanden sind; und
eine wesentliche Anzahl an Fasern in Regionen (R) mit geringer Konzentration in der Ebene des Fasermatten- bzw. Vlieskörpers (16) straff bzw. straff gespannt ist und sich in verschiedene Richtungen erstreckt, die sich von den Basen (B) der Schlingen (14) strahlenförmig ausbreiten.

2. Schlingen aufweisende Befestigungskomponente nach Anspruch 1, **dadurch gekennzeichnet, dass** die Basen (B) der Schlingen (14) straff gespannte Abschnitte der Fasern des Fasermatten- bzw. Vlieskörpers enthalten, die als schlingenbildende Fasern ausgebildet sind.

3. Schlingen aufweisende Befestigungskomponente nach Anspruch 2, **dadurch gekennzeichnet, dass** die straff gespannten Fasern der Basen (B) zur Definition bzw. Abgrenzung von freistehenden Formationen bzw. Gebilden (20) beitragen, die sich von der Ebene des Fasermatten- bzw. Vlieskörpers (16) erstrecken und einhakbare Schlingen (14) enthalten.

4. Schlingen aufweisende Befestigungskomponente nach Anspruch 3, **dadurch gekennzeichnet, dass** die freistehenden Formationen bzw. Gebilde (20) verlängerte Rümpfe bzw. Stämme (22) umfassen, von denen sich mehrere einhakbare Schlingen (14) erstrecken.

5. Schlingen aufweisende Befestigungskomponente nach einem der vorangegangenen Ansprüche, **dadurch gekennzeichnet, dass** die relativ hohe Konzentration an Fasern an den Basen (B) der einhakbaren Schlingen gestraffte bzw. verstärkte Faserverwicklungen bzw. -verknäulungen definiert bzw. abgrenzt.

6. Schlingen aufweisende Befestigungskomponente nach einem der vorangegangenen Ansprüche, **dadurch gekennzeichnet, dass** das kombinierte Gewicht des Fasermatten- bzw. Vlieskörpers (16) und den Schlingen (14) kleiner ist als 2 Unzen/Quadratyard (68g/m²).

7. Schlingen aufweisende Befestigungskomponente nach einem der vorangegangenen Ansprüche, **dadurch gekennzeichnet, dass** die Schlingen (14) in relativ zufälligem Muster über der Fläche des Fasermatten- bzw. Vlieskörpers (16) angeordnet sind.

8. Schlingen aufweisende Befestigungskomponente nach einem der vorangegangenen Ansprüche, **dadurch gekennzeichnet, dass** ein verfestigtes Fluid- bzw. Flüssigkeitsbindemittel (21) an den Basen (B) der Schlingen konzentriert ist.

9. Schlingen aufweisende Befestigungskomponente nach Anspruch 8, **dadurch gekennzeichnet, dass** das verfestigte Flüssigkeitsbindemittel (21) aus der Gruppe bestehend aus Acrylharzen, Urethanen, Polyvinylen, Formaldehyen, Glyoxalen und Epoxiden ausgewählt ist.

10. Schlingen aufweisende Befestigungskomponente nach Anspruch 8 oder 9, **dadurch gekennzeichnet, dass** das verfestigte Flüssigkeitsbindemittel (21) an den Basen (B) der Schlingen mindestens partiell als Ergebnis der Kapillarströmung vor der Verfestigung konfiguriert ist.

11. Schlingen aufweisende Befestigungskomponente nach einem der Ansprüche 8-10, **dadurch gekennzeichnet, dass** das verfestigte Flüssigkeitsbindemittel (21) etwa zwischen 20 und 40% des Gesamtgewichts, vorzugsweise etwa ein Drittel des Gesamtgewichts der Fasermatte bzw. des Vlies (12), einschließlich der Schlingen (14), ausmacht.

12. Schlingen aufweisende Befestigungskomponente nach einem der Ansprüche 1 bis 7, kombiniert mit einer Schicht (302) aus thermoplastischem Material, einer zweiten Fläche des Fasermatten- bzw. Vlieskörpers (16), die der Fläche gegenüberliegt bzw. entgegengesetzt ist, von der sich die einhakbaren Schlingen (14) erstrecken, die in die Schicht aus thermoplastischem Material eingebettet bzw. eingekapselt sind.

13. Schlingen aufweisende Befestigungskomponente nach Anspruch 12, **dadurch gekennzeichnet, dass** Hakenelemente (304) auf einer Oberfläche der Schicht (302) aus thermoplastischem Material integral geformt sind.

14. Schlingen aufweisende Befestigungskomponente nach einem der vorangegangenen Ansprüche, **dadurch gekennzeichnet, dass** die thermoplastischen Fasern der Fasermatte bzw. des Vlies (12) an den Basen (B) der einhakbaren Schlingen (14) warmverschweißt sind.

15. Schlingen aufweisende Befestigungskomponente nach einem der vorangegangenen Ansprüche, die aus einer genadelten nichtgewebten Ausgangsfasermatte (160) vorgegebenen Ausmaßes bzw. vorgegebener Größe gebildet ist, **dadurch gekennzeichnet, dass** die Fasermatte bzw. das Vlies (12) der Schlingen aufweisenden Befestigungskomponente in ihrer bzw. seiner Erstreckungsebene in stabilisiertem gedehnten bzw. gezogenen Zustand eine mindestens 20% größere Fläche, vorzugsweise mindestens 50% größere Fläche und noch bevorzugter mindestens 100% größere Fläche aufweist als die Fläche der Ausgangsfasermatte (160).

16. Schlingen aufweisende Befestigungskomponente nach Anspruch 15, **dadurch gekennzeichnet, dass** die Fasermatte bzw. das Vlies (12) in gedehntem bzw. gezogenem Zustand mindestens 20% in mindestens eine erste Richtung in der allgemeinen Erstreckungsebene der Fasermatte bzw. des Vlies (12) verläuft.

17. Schlingen aufweisende Befestigungskomponente nach Anspruch 15, **dadurch gekennzeichnet, dass** die Fasermatte bzw. das Vlies (12) in gedehntem bzw. gezogenem Zustand mindestens 20% in jede der zwei orthogonalen Richtungen in der allgemeinen Erstreckungsebene der Fasermatte bzw. des Vlies verläuft.

18. Schlingen aufweisende Befestigungskomponente nach einem der Ansprüche 15 bis 17, **dadurch gekennzeichnet, dass** die Ausgangsfasermatte (160) Schichten aus im Allgemeinen parallelen Fasern umfasst mit Fasern aus einigen Schichten, die hinsichtlich der Fasern anderer Schichten in spitzen Winkeln ausgerichtet sind, **dadurch gekennzeichnet, dass**, im Ergebnis des Dehnens bzw. Ziehens die spitzen Winkel zwischen den Fasern des Fasermatten- bzw. Vlieskörpers (16), die den verschiedenen Schichten der Ausgangsfasermatte (160) entsprechen, im Allgemeinen größer sind als die entsprechenden Winkel in der Ausgangsfasermatte (160), während zumindest viele der Fasern, die jeder Schicht entsprechen, relativ parallel verbleiben.

19. Schlingen aufweisende Befestigungskomponente nach Anspruch 18, **dadurch gekennzeichnet, dass** sich die Schichten einer sich in Längsrichtung erstreckenden Ausgangsfasermatte (160) vorzugsweise schräg über die Fasermatte erstrecken, **dadurch gekennzeichnet, dass**, im Ergebnis des Dehnens bzw. Ziehens der Ausgangsfasermatte (160) in Längsrichtung, die spitzen Winkel zwischen den Fasern des Fasermatten- bzw. Vlieskörpers (16), die den verschiedenen Schichten der Ausgangsfasermatte (160) entsprechen, im Allgemeinen größer sind als die entsprechenden Winkel in der Ausgangsfasermatte (160).

20. Schlingen aufweisende Befestigungskomponente nach einem der vorangegangenen Ansprüche mit einer Gesamtdicke (t), einschließlich des Fasermatten- bzw. Vlieskörpers (16) und einer Mehrheit der Schlingen (14), von weniger als 0,150 Zoll (4,0mm), vorzugsweise weniger als 0,100 Zoll (2,5mm).

21. Schlingen aufweisende Befestigungskomponente nach einem der vorangegangenen Ansprüche, **dadurch gekennzeichnet, dass** sich die einhakbaren Schlingen (14) von den zugehörigen Schlingenbasen (B) in die Ebene der Fasermatte bzw. des Vlies zu einer durchschnittlichen Schlingenhöhe (hL), gemessen als senkrechter Abstand vom Fasermatten- bzw. Vlieskörper (16), von etwa zwischen 0,020 und 0,060 Zoll (0,5 und 1,0mm) erstrecken.

22. Schlingen aufweisende Befestigungskomponente nach einem der vorangegangenen Ansprüche, **dadurch gekennzeichnet, dass** die Fasermatte bzw. das Vlies eine Gesamtdicke (t), einschließlich des Fasermatten- bzw. Vlieskörpers (16) und einer Mehrheit der Schlingen (14), aufweist und wobei die durchschnittliche Schlingenhöhe (hL) zwischen 0,5 und 0,8 mal so groß ist wie die Gesamtdicke (t) des Produktes.

23. Schlingen aufweisende Befestigungskomponente nach einem der vorangegangenen Ansprüche mit etwa zwischen 50 und 1000 gestrafften bzw. verstärkten Faserverwicklungen bzw. -verknäulungen pro Quadratzoll (8 und 160/cm²) der Fasermatte- bzw. Vliesfläche, von der sich die einhakbaren Schlingen (14) erstrecken.

24. Schlingen aufweisende Befestigungskomponente nach einem der vorangegangenen Ansprüche, **dadurch gekennzeichnet, dass** die Fasermatte bzw. das Vlies (12) im Allgemeinen aus Fasern besteht, die eine Festigkeit von mindestens 2,8g/den, vorzugsweise mindestens 5,0g/den und noch bevorzugter mindestens 8,0g/den aufweisen.

25. Schlingen aufweisende Befestigungskomponente nach einem der vorangegangenen Ansprüche, **dadurch gekennzeichnet, dass** sich die Schlingen (14) vom Fasermatten- bzw. Vlieskörper (16) bis zu verschiedenen Höhen (hL) erstrecken, um eine mehrstufige Anordnung aus einhakbaren Schlingen (14) zu bilden.

26. Schlingen aufweisende Befestigungskomponente nach einem der vorangegangenen Ansprüche mit einer Gurley-Steifheit von weniger als 300mg.

27. Schlingen aufweisende Befestigungskomponente nach einem der vorangegangenen Ansprüche einschließlich getrennter Versteifungsstränge (28), die sich in mindestens eine Richtung innerhalb der Ebene des Fasermatten- bzw. Vlieskörpers (16) erstrecken.

28. Verpackung (56), die einen ersten Verschlussabschnitt (60) umfasst, welcher Schlingen aufweist, sowie einen zweiten Verschlussabschnitt (58), welcher Haken aufweist, die konstruiert sind, um in die Schlingen des ersten Verschlussabschnitts einzuhaken, um die Verpackung (56) in einer geöffneten oder geschlossenen Position zu halten, wobei die Verpackung **dadurch gekennzeichnet ist, dass** der erste Verschlussabschnitt (60) das Schlingen aufweisende Befestigungsprodukt nach einem der vorangegangenen Ansprüche umfasst.

29. Verpackung nach Anspruch 28, **dadurch gekennzeichnet, dass** der erste und zweite Verschlussabschnitt (58 und 60) verbunden und gelöst eine kombinierte Gesamtdicke von weniger als ca. 0,075 Zoll (1,9mm) aufweisen.

30. Einwegkleidungsstück (50, 54), das einen Stoff und einen Verschluss mit Haken aufweist, die angeordnet sind, um in die Schlingen des Stoffs einzuhaken, um einen lösbaren Verschluss zu bilden, um das Kleidungsstück (50, 54) auf dem Träger zu fixieren, wobei das Kleidungsstück **dadurch gekennzeichnet ist, dass** der Stoff die Schlingen aufweisende Befestigungskomponente nach einem der Ansprüche 1 bis 27 umfasst.

31. Luftfilter (80), **dadurch gekennzeichnet, dass** der Filter das Schlingen aufweisende Befestigungsprodukt nach einem der Ansprüche 1 bis 27 umfasst und angeordnet ist, um eine Luftströmung abzufangen und zu filtern.

32. Geotextile Barriere (352), **dadurch gekennzeichnet, dass** die Barriere das Schlingen aufweisende Befestigungsprodukt nach einem der Ansprüche 1 bis 27 umfasst und angeordnet ist, um eine Grundwasserströmung abzufangen und zu filtern.

33. Geotextile Barriere (352), **dadurch gekennzeichnet, dass** die Barriere das Schlingen aufweisende Befestigungsprodukt nach einem der Ansprüche 1 bis 27 umfasst und angeordnet ist, um eine Bodenschichtgrenze zu stabilisieren.

34. Verschluss- bzw. Befestigungsprodukt mit einer plattenförmigen Polymerbasis (302) mit Haken (304), die auf eine Seite der Basis integral geformt sind und sich von einer Seite der Basis erstrecken, und einem schlingenartigen Material, das dauerhaft an eine gegenüberliegende bzw. entgegengesetzte Seite der Basis gebunden ist und sich über sie erstreckt, wobei das Produkt **dadurch gekennzeichnet ist, dass** das schlingenartige Material das Schlingen aufweisende Material (12) nach einem der Ansprüche 1 bis 27 umfasst und die Schlingen (14) des Schlingen aufweisenden Materials (12) adaptiert sind, um von den Haken (304) des Produktes eingehakt zu werden.

35. Kombination aus
einem Haken aufweisenden Befestigungsprodukt (10), das Haken aufweist, welche sich von einer Oberfläche davon erstrecken; und
dem Schlingen aufweisenden Befestigungsprodukt nach einem der Ansprüche 1 bis 27, wobei die Haken des Haken aufweisenden Befestigungsproduktes adaptiert sind, um die Schlingen (14) des Schlingen aufweisenden Befestigungsproduktes einzuhaken, um einen lösbaren Verschluss zu bilden.

36. Kombination nach Anspruch 35, **dadurch gekennzeichnet, dass** das Haken aufweisende Befestigungsprodukt (10) und das Schlingen aufweisende Befestigungsprodukt verbunden und gelöst eine kombinierte Gesamtdicke von weniger als etwa 0,075 Zoll (1,9mm), vorzugsweise weniger als etwa 0,050 Zoll (1,27mm) aufweisen.

37. Verfahren zum Bilden einer Schlingen bzw. Schlaufen aufweisenden Befestigungskomponente für Klettverschlüsse aus einer im Allgemeinen ebenen nichtgewebten Fasermatte (160) aus verwickelten bzw. verknäuelten Fasern, wobei das Verfahren **gekennzeichnet ist, durch**
Dehnen bzw. Ziehen der Fasermatte (160) in mindestens eine Richtung, wodurch eine gedehnte bzw. gezogene Fasermatte bzw. ein Vlies (12) erzeugt wird, die bzw. das einen im Allgemeinen ebenen Fasermatten- bzw. Vlieskörper (16) mit einer uneinheitlichen Verteilung der Fasern aufweist, wobei die Fasern in relativ hoher Konzentration in den Regionen der Basen (B) der einhakbaren Schlingen (14) vorhanden sind, die sich vom Fasermatten- bzw. Vlieskörper (16) erstrecken, und in relativ geringer Konzentration in Regionen (R) der Fasermatte bzw. des Vlies (12) zwischen den Basen (B) der Schlingen, wobei
eine wesentliche Anzahl an Fasern in Regionen (R) von geringer Konzentration in der Ebene des Fasermatten- bzw. Vlieskörpers (16) straff bzw. straff gespannt sind und sich in verschiedene Richtungen erstrecken, die sich von den Basen (B) der Schlingen (14) strahlenförmig ausbreiten; und
Stabilisieren der Fasermatte bzw. des Vlies (12) in ihrem bzw. seinem gedehnten bzw. gezogenen Zustand.

38. Verfahren nach Anspruch 37, **dadurch gekennzeichnet, dass** die Fasermatte bzw. das Vlies (12) in ihrem bzw. seinem gedehnten bzw. gezogenen Zustand stabilisiert ist durch das Verfestigen eines Fluid- bzw. Flüssigkeitsbindemittels (21), das unter Bedingungen auf den Fasermatten- bzw. Vlieskörper (16) angewendet wird, die das Bindemittel an den Basen (B) der Schlingen konzentrieren.

39. Verfahren nach Anspruch 37, **dadurch gekennzeichnet, dass** das Gewicht der Fasermatte bzw. des Vlies (12) geringer ist als 4 Unzen/Quadratyard (135g/m²).

40. Verfahren nach Anspruch 39, **dadurch gekennzeichnet, dass** das Dehnen bzw. Ziehen bewirkt, dass die straff gespannten Fasern des Fasermatten- bzw. Vlieskörpers (16) als schlingenbildende Fasern in den Basen (B) der Schlingen (14) ausgebildet sind.

41. Verfahren nach einem der vorangegangenen Ansprüche, **dadurch gekennzeichnet, dass** die Fasermatte auf eine Weise gedehnt bzw. gezogen wird, dass die relativ hohe Konzentration von Fasern an den Basen (B) der einhakbaren Schlingen gestraffte bzw. verstärkte Faserverwicklungen bzw. -verknäulungen definiert bzw. abgrenzt.

42. Verfahren nach einem der vorangegangenen Ansprüche, **dadurch gekennzeichnet, dass** die Ausgangsfasermatte gedehnt bzw. gezogen wird, um eine Fasermatte bzw. ein Vlies (12) zu erzeugen, die bzw. das ein Gewicht, einschließlich der Schlingen (14), von weniger als 2 Unzen/Quadratyard (68g/m²) aufweist.

43. Verfahren nach einem der vorangegangenen Ansprüche, **dadurch gekennzeichnet, dass** die Fasermatte bzw. das Vlies (12) in ihrem bzw. seinem gedehnten bzw. gezogenen Zustand stabilisiert ist durch das Verfestigen eines Fluid- bzw. Flüssigkeitsbindemittels (21), das unter Bedingungen auf den Fasermatten- bzw. Vlieskörper (16) angewendet wird, die das Bindemittel an den Basen (B) der Schlingen konzentrieren.

44. Verfahren nach Anspruch 43, **dadurch gekennzeichnet, dass** das Flüssigkeitsbindemittel (21) zumindest vor einem Teil des Dehnens bzw. Ziehens angewendet wird.

45. Verfahren nach Anspruch 43, **dadurch gekennzeichnet, dass** eine ausgewählte Menge an Bindemittel (21) angewendet wird, so dass das Bindemittel etwa zwischen 20 und 40% des Gesamtgewichts ausmacht, vorzugsweise ein Drittel des Gesamtgewichts der Fasermatte bzw. des Vlies (12), einschließlich der Schlingen (14).

46. Verfahren nach einem der vorangegangenen Ansprüche, **dadurch gekennzeichnet, dass** die Fasermatte (160) mit darin eingebetteten schmelzbaren thermoplastischen Fasern versehen ist und die gedehnte bzw. gezogene Fasermatte bzw. das gedehnte bzw. gezogene Vlies unter Bedingungen stabilisiert ist, die bewirken, dass die schmelzbaren thermoplastischen Fasern an den Basen (B) der einhakbaren Schlingen warmverschweißt werden.

47. Verfahren nach einem der vorangegangenen Ansprüche, **dadurch gekennzeichnet, dass** die Fasermatte bzw. das Vlies (12) in einem gedehnten bzw. gezogenen Zustand stabilisiert ist, in dem die Fasermatte bzw. das Vlies in ihrer bzw. seiner allgemeinen Erstreckungsebene eine mindestens 20% größere Fläche, vorzugsweise mindestens 50% größere Fläche und noch bevorzugter mindestens 100% größere Fläche aufweist als die Fläche der Fasermatte (160) vor dem Dehnen bzw. Ziehen.

48. Verfahren nach Anspruch 47, **dadurch gekennzeichnet, dass** die Fasermatte (160) mindestens 20% in eine erste Richtung in der allgemeinen Erstreckungsebene des Fasermatten- bzw. des Vlieskörpers (16) gedehnt bzw. gezogen wird.

49. Verfahren nach Anspruch 47, **dadurch gekennzeichnet, dass** die Fasermatte (160) mindestens 20% in jede der zwei orthogonalen Richtungen in der allgemeinen Erstreckungsebene des Fasermatten- bzw. des Vlieskörpers (16) gedehnt bzw. gezogen wird.

50. Verfahren zum Filtern von Luft, **gekennzeichnet durch** Anordnen der Fasermatte bzw. des Vlies (12) des Schlingen aufweisenden Befestigungsproduktes nach einem der Ansprüche 1 bis 27, um eine zu filternde Luftströmung abzufangen.

51. Verfahren zum Filtern von Grundwasser, **gekennzeichnet durch** Anordnen der Fasermatte bzw. des Vlies (12) des Schlingen aufweisenden Befestigungsproduktes nach einem der Ansprüche 1 bis 27, um eine zu filternde Grundwasserströmung abzufangen und **durch** in Position halten der Fasermatte bzw. des Vlies **durch** Einhaken der Schlingen (14) des Schlingen aufweisenden Befestigungsproduktes mit einer Länge des Haken aufweisenden Befestigungsbandes (360).

52. Verfahren zum Stabilisieren von Boden, **gekennzeichnet durch** Anordnen der Fasermatte bzw. des Vlies (12) des Schlingen aufweisenden Befestigungsproduktes nach einem der Ansprüche 1 bis 27 zwischen angrenzende bzw. benachbarte Schichten von zu stabilisierendem Boden, und **durch** in Position halten der Fasermatte bzw. des Vlies **durch** Einhaken der Schlingen (14) des Schlingen aufweisenden Befestigungsproduktes mit einer Länge des Haken aufweisenden Befestigungsbandes (360).

## Revendications

1. Composant de fixation à bouclettes d'une attache à crochets et à bouclettes, comprenant un tissu non tissé (12) de fibres emmêlées, ayant un corps en tissu généralement plan (16), des fibres s'étendant depuis au moins une face dudit corps sous la forme de bouclettes (14) pouvant être engagées dans des crochets, **caractérisé en ce que** :
- le tissu (12), y compris le corps en tissu (16) et les bouclettes (14), a un poids inférieur à environ 4 onces par yard carré (135 grammes par mètre carré) ;
- le corps en tissu comprend une répartition non uniforme de fibres dans laquelle des fibres sont dans des concentrations relativement hautes dans les régions des bases (B) des bouclettes (14) pouvant être engagées dans des crochets, et dans des concentrations relativement plus basses dans des régions (R) disposées entre les bases (B) des bouclettes ; et
- un nombre important de fibres dans des régions (R) de plus basse concentration sont tendues dans le plan du corps en tissu (16) et s'étendent dans différentes directions en rayonnant depuis les bases (B) des bouclettes (14).

2. Composant de fixation à bouclettes selon la revendication 1, **caractérisé en ce que** les bases (B) des bouclettes (14) contiennent des parties tendues de fibres du corps en tissu, lesquelles sont traînées autour des fibres formant des boucles.

3. Composant de fixation à bouclettes selon la revendication 2, **caractérisé en ce que** des fibres tendues des bases (B) contribuent à définir des formations faisant saillie (20) qui s'étendent depuis le plan du corps en tissu (16) et qui contiennent les bouclettes (14) pouvant être engagées dans des crochets.

4. Composant de fixation à bouclettes selon la revendication 3, **caractérisé en ce que** les formations faisant saillie (20) comprennent des troncs allongés (22), des bouclettes (14) pouvant être engagées dans des crochets s'étendant depuis chacun desdits troncs.

5. Composant de fixation à bouclettes selon l'une quelconque des revendications précédentes, **caractérisé en ce que** les concentrations relativement hautes de fibres sur les bases (B) des bouclettes pouvant être engagées dans des crochets définissent des emmêlements denses de fibres.

6. Composant de fixation à bouclettes selon l'une quelconque des revendications précédentes, **caractérisé en ce que** le poids combiné du corps en tissu (16) et des bouclettes (14) est inférieur à environ 2 onces par yard carré (68 grammes par mètre carré).

7. Composant de fixation à bouclettes selon l'une quelconque des revendications précédentes, **caractérisé en ce que** les bouclettes (14) sont disposées d'un côté à un autre du corps en tissu (16) dans un schéma relativement aléatoire.

8. Composant de fixation à bouclettes selon l'une quelconque des revendications précédentes, **caractérisé en ce qu'**un liant fluide solidifié (21) est concentré sur les bases (B) des bouclettes.

9. Composant de fixation à bouclettes selon la revendication 8, **caractérisé en ce que** le liant fluide solidifié (21) est sélectionné depuis le groupe constitué d'acryliques, d'uréthanes, de polyvinyles, de formaldéhydes, de glyoxal et d'époxydes.

10. Composant de fixation à bouclettes selon l'une ou l'autre des revendications 8 et 9, **caractérisé en ce que** le liant fluide solidifié (21) est configuré sur les bases (B) des bouclettes au moins partiellement comme le résultat d'un flux capillaire avant la solidification.

11. Composant de fixation à bouclettes selon l'une quelconque des revendications 8 à 10, **caractérisé en ce que** le liant fluide solidifié (21) représente entre environ 20 et 40 pour cent du poids total, de préférence environ un tiers du poids total du tissu (12), y compris les bouclettes (14).

12. Composant de fixation à bouclettes selon l'une quelconque des revendications 1 à 7, combiné à une couche (302) de matériau thermoplastique, dans lequel une seconde face du corps en tissu (16) étant opposée à la face depuis laquelle s'étendent les bouclettes (14) pouvant être engagées dans des crochets, est encapsulée dans la couche de matériau thermoplastique.

13. Composant de fixation à bouclettes selon la revendication 12, **caractérisé en ce que** des éléments à crochets (304) sont moulés intégralement sur une surface de la couche (302) de matériau thermoplastique.

14. Composant de fixation à bouclettes selon l'une quelconque des revendications précédentes, **caractérisé en ce que** des fibres thermoplastiques du tissu (12) sont thermo-soudées sur les bases (B) des bouclettes (14) pouvant être engagées dans des crochets.

15. Composant de fixation à bouclettes selon l'une quelconque des revendications précédentes, formé à partir d'une batte de départ aiguilletée non tissée (160) d'une dimension donnée, **caractérisé en ce que** le tissu (12) du composant de fixation à bouclettes est, dans son plan général d'extension, dans un état étiré stabilisé d'une surface d'au moins 20 pour cent plus grande, de préférence d'une surface d'au moins 50 pour cent plus grande, et de manière encore plus préférentielle d'une surface d'au moins 100 pour cent plus grande que la surface de la batte de départ (160).

16. Composant de fixation à bouclettes selon la revendication 15, **caractérisé en ce que** le tissu (12) est dans un état étiré d'au moins 20 pour cent dans au moins une première direction dans le plan général d'extension du tissu (12).

17. Composant de fixation à bouclettes selon la revendication 15, **caractérisé en ce que** le tissu (12) est dans un état étiré d'au moins 20 pour cent dans chacune des deux directions orthogonales dans le plan général d'extension du tissu.

18. Composant de fixation à bouclettes selon l'une quelconque des revendications 15 à 17, **caractérisé en ce que** la batte de départ (160) comprend des couches de fibres généralement parallèles, des fibres de certaines couches étant orientées selon des angles aigus par rapport aux fibres d'autres couches, et **caractérisé en ce que**, comme un résultat de l'étirement, les angles aigus entre des fibres du corps en tissu (16) qui correspondent aux différentes couches de la batte de départ (160) sont généralement plus grands que des angles correspondants dans la batte de départ (160), tandis qu'au moins un grand nombre de fibres qui correspondent à chaque couche restent relativement parallèles.

19. Composant de fixation à bouclettes selon la revendication 18, **caractérisé en ce que** les couches d'une batte de départ (160) s'étendant longitudinalement s'étendent de manière prédominante transversalement d'un côté à l'autre de la batte, et **caractérisé en ce que**, comme un résultat de l'étirement longitudinal de la batte de départ (160), les angles aigus entre des fibres du corps en tissu (16) qui correspondent aux différentes couches de la batte de départ (160) sont généralement plus grands que des angles correspondants dans la batte de départ (160).

20. Composant de fixation à bouclettes selon l'une quelconque des revendications précédentes, ayant une épaisseur hors tout (t), y compris le corps en tissu (16) et une majorité des bouclettes (14), inférieure à environ 0,150 pouce (4,0 mm), de préférence inférieure à environ 0,100 pouce (2,5 mm).

21. Composant de fixation à bouclettes selon l'une quelconque des revendications précédentes, **caractérisé en ce que** les bouclettes (14) pouvant être engagées dans des crochets s'étendent depuis les bases (B) de bouclettes associées à l'intérieur du plan du tissu à une hauteur de bouclette moyenne (hL) entre environ 0,020 et 0,060 pouce (0,5 et 1,0 mm), mesurée comme la distance perpendiculaire depuis le corps en tissu (16).

22. Composant de fixation à bouclettes selon l'une quelconque des revendications précédentes, **caractérisé en ce que** le tissu (12) a une épaisseur hors tout (t), y compris le corps en tissu (16) et une majorité des bouclettes (14), et dans le quel la hauteur de bouclette moyenne (hL) est environ entre 0,5 et 0,8 fois plus épaisse que l'épaisseur hors tout (t) du produit.

23. Composant de fixation à bouclettes selon l'une quelconque des revendications précédentes, ayant entre environ 50 et 1000 emmêlements de fibres denses par pouce carré (8 et 160 par centimètre carré) de surface de tissu, depuis lesquels s'étendent des bouclettes (14) pouvant être engagées dans des crochets.

24. Composant de fixation à bouclettes selon l'une quelconque des revendications précédentes, **caractérisé en ce que** le tissu (12) est généralement composé de fibres ayant une ténacité d'au moins 2,8 grammes par denier, de préférence d'au moins 5,0 grammes par denier, et de manière encore plus préférentielle d'au moins 8,0 grammes par denier.

25. Composant de fixation à bouclettes selon l'une quelconque des revendications précédentes, **caractérisé en ce que** les bouclettes (14) s'étendent depuis le corps en tissu (16) à des hauteurs variées (h_{L}) pour former une disposition à plusieurs niveaux de bouclettes (14) pouvant être engagées dans des crochets.

26. Composant de fixation à bouclettes selon l'une quelconque des revendications précédentes, ayant une raideur de Gurley inférieure à environ 300 milligrammes.

27. Composant de fixation à bouclettes selon l'une quelconque des revendications précédentes, comprenant des cordons de consolidation séparés (28) qui s'étendent au moins dans une direction à l'intérieur du plan du corps en tissu (16).

28. Boîtier (56), comprenant une première partie de fermeture (60) ayant des bouclettes et une seconde partie de fermeture (58) ayant des crochets construits pour engager les bouclettes de la première partie de fermeture afin de maintenir le boîtier (56) dans une position ouverte ou fermée, le boîtier étant **caractérisé en ce que** la première partie de fermeture (60) comprend le produit de fixation à bouclettes selon l'une quelconque des revendications précédentes.

29. Boîtier selon la revendication 28, **caractérisé en ce que** la première et la seconde partie de fermeture (58 et 60) ont une épaisseur hors tout combinée, engagée et au repos, inférieure à environ 0,075 pouce (1,9 mm).

30. Article de vêtement à usage unique (50, 54), ayant une étoffe et une fixation avec des éléments à crochets disposés pour engager des bouclettes de l'étoffe afin de former une attache détachable pour maintenir l'article de vêtement (50, 54) sur la personne qui en est revêtue, l'article de vêtement étant **caractérisé en ce que** l'étoffe comprend le composant de fixation à bouclettes selon l'une quelconque des revendications 1 à 27.

31. Filtre à air (80), **caractérisé en ce que** le filtre comprend le produit de fixation à bouclettes selon l'une quelconque des revendications 1 à 27, lequel est disposé pour intercepter et filtrer un flux d'air.

32. Barrière géotextile (352), **caractérisée en ce que** la barrière comprend le produit de fixation à bouclettes selon l'une quelconque des revendications 1 à 27, lequel est disposé pour intercepter et filtrer un flux de la nappe phréatique.

33. Barrière géotextile (352), **caractérisée en ce que** la barrière comprend le produit de fixation à bouclettes selon l'une quelconque des revendications 1 à 27, lequel est disposé pour stabiliser une limite de couche dans le sol.

34. Produit de fixation, ayant une base polymère en forme de feuille (302) et comprenant des crochets (304) moulés intégralement avec et s'étendant depuis un côté de la base, et un matériau à bouclettes lié de manière permanente à et s'étendant sur un côté opposé de la base, le produit étant **caractérisé en ce que** le matériau à bouclettes du produit comprend le matériau de fixation à bouclettes (12) selon l'une quelconque des revendications 1 à 27, et **en ce que** les bouclettes (14) du matériau de fixation à bouclettes (12) sont adaptées pour être engagées par les crochets (304) du produit.

35. En combinaison,
- un produit de fixation à crochets (10) ayant des crochets s'étendant depuis une surface de celui-ci ; et
- le produit de fixation à bouclettes selon l'une quelconque des revendications 1 à 27, les crochets du produit de fixation à crochets étant adapté pour engager les bouclettes (14) du produit de fixation à bouclettes afin de former une attache détachable.

36. Combinaison selon la revendication 35, **caractérisée en ce que** le produit de fixation à crochets (10) et le produit de fixation à bouclettes ont une épaisseur hors tout combinée, engagée et au repos, inférieure à environ 0,075 pouce (1,9 mm), de préférence inférieure à environ 0,050 pouce (1,27 mm).

37. Méthode de formage d'un composant de fixation à bouclettes pour la fixation par des crochets et des bouclettes depuis une batte non tissée généralement plane (160) de fibres emmêlées, la méthode étant **caractérisée par** :
- l'étirement de la batte (160) dans au moins une direction, produisant de cette manière un tissu étiré (12) ayant un corps en tissu généralement plan (16) et présentant une répartition non uniforme de fibres, comportant des fibres dans des concentrations relativement hautes dans des régions de bases (B) de bouclettes (14) pouvant être engagées dans des crochets s'étendant depuis le corps en tissu (16) ainsi que des fibres dans des concentrations relativement plus basses des régions (R) du tissu entre les bases (B) des bouclettes, comprenant :
- un nombre important de fibres dans des régions (R) de plus basse concentration qui sont tendues dans le plan du corps en tissu (16) et qui s'étendent dans différentes directions en rayonnant depuis les bases (B) des bouclettes (14) ; et
- la stabilisation du tissu (12) dans son état étiré.

38. Méthode selon la revendication 37, **caractérisée en ce que** le tissu (12) est stabilisé dans son état étiré par la solidification d'un liant fluide (21) appliqué sur le corps en tissu (16) dans des conditions qui concentrent le liant sur les bases (B) des bouclettes.

39. Méthode selon la revendication 37, **caractérisée en ce que** le tissu (12) est d'un poids inférieur à environ 4 onces par yard carré (135 grammes par mètre carré).

40. Méthode selon la revendication 39, **caractérisée en ce que** l'étirement provoque que des fibres tendues du corps en tissu soient traînées autour des fibres formant des boucles dans les bases (B) des bouclettes (14).

41. Méthode selon l'une quelconque des revendications de méthode précédentes, **caractérisée en ce que** la batte est étirée d'une manière telle que les concentrations relativement hautes de fibres sur les bases (B) des bouclettes pouvant être engagées dans des crochets définissent des emmêlements de fibres denses.

42. Méthode selon l'une quelconque des revendications de méthode précédentes, **caractérisée en ce que** la batte est étirée de manière à produire un tissu (12) ayant un poids, y compris les boucles (14), inférieur à environ 2 onces par yard carré (68 grammes par mètre carré).

43. Méthode selon l'une quelconque des revendications de méthode précédentes, **caractérisée en ce que** le tissu (12) est stabilisé dans son état étiré par la solidification d'un liant fluide (21) appliqué sur le corps en tissu (16) dans des conditions qui concentrent le liant sur les bases (B) des bouclettes.

44. Méthode selon la revendication 43, **caractérisée en ce que** le liant fluide (21) est appliqué avant au moins une partie de l'étirement.

45. Méthode selon la revendication 43, **caractérisée en ce qu'**une quantité sélectionnée de liant (21) est appliquée de telle sorte que le liant représente entre environ 20 et 40 pour cent du poids total, de préférence environ un tiers du poids total du tissu (12), y compris les bouclettes (14).

46. Méthode selon l'une quelconque des revendications de méthode précédentes, **caractérisée en ce que** la batte (160) est prévue avec des fibres thermoplastiques fusibles noyées dedans, et **en ce que** le tissu étiré est stabilisé dans des conditions qui provoquent que les fibres thermoplastiques fusibles soient thermo-soudées sur les bases (B) des bouclettes (14) pouvant être engagées dans des crochets.

47. Méthode selon l'une quelconque des revendications de méthode précédentes, **caractérisée en ce que** le tissu (12) est stabilisé dans un état étiré dans lequel le tissu a, dans son plan général d'extension, a une surface d'au moins 20 pour cent plus grande, de préférence d'une surface d'au moins 50 pour cent plus grande, et de manière encore plus préférentielle d'une surface d'au moins 100 pour cent plus grande que la surface de la batte de départ (160) avant l'étirement.

48. Méthode selon la revendication 47, **caractérisée en ce que** la batte (160) est étirée d'au moins 20 pour cent dans une première direction dans le plan général d'extension du corps en tissu (16).

49. Méthode selon la revendication 47, **caractérisée en ce que** la batte (160) est étirée d'au moins 20 pour cent dans chacune des deux directions orthogonales dans le plan général d'extension du corps en tissu (16).

50. Méthode de filtrage de l'air, **caractérisée par** la disposition du tissu (12) du produit de fixation à bouclettes selon l'une quelconque des revendications 1 à 27 pour intercepter un flux d'air qui doit être filtré.

51. Méthode de filtrage de la nappe phréatique, **caractérisée par** la disposition du tissu (12) du produit de fixation à bouclettes selon l'une quelconque des revendications 1 à 27 pour intercepter un flux de la nappe phréatique, lequel doit être filtré, et par la retenue du tissu dans une position en engageant les bouclettes (14) du produit de fixation à bouclettes avec une longueur de bande de fixation à crochets (360).

52. Méthode de stabilisation du sol, **caractérisée par** la disposition du tissu (12) du produit de fixation à bouclettes selon l'une quelconque des revendications 1 à 27 entre des couches adjacentes du sol, lesquelles doivent être stabilisées, et par la retenue du tissu dans une position en engageant les bouclettes (14) du produit de fixation à bouclettes avec une longueur de bande de fixation à crochets (360).
